# EUROPEAN PATENT SPECIFICATION

(11) **EP 1 833 551 B1**
(45) Date of publication and mention of the grant of the patent: **27.02.2013**
(21) Application number: 05855382.7
(22) Date of filing: 22.12.2005
(51) Int. Cl.: A61N 1/05, A61N 1/375

(54) **IMPLANTABLE ADDRESSABLE SEGMENTED ELECTRODES**
IMPLANTIERBARE ADRESSIERBARE SEGMENTIERTE ELEKTRODEN
ELECTRODES SEGMENTEES ADRESSABLES IMPLANTABLES

(30) Priority: 22.12.2004 US 638692 P; 22.02.2005 US 655609 P; 15.12.2005 US 751111 P; 20.12.2005 US 752733 P
(43) Date of publication of application: 19.09.2007
(73) Proprietor: Proteus Digital Health, Inc., Redwood City CA 94065 (US)
(72) Inventor: JENSEN, Marc, Los Gatos, California 95032 (US); COSTELLO, Benedict, J., Berkeley, California 94730 (US); THOMPSON, Todd, San Jose, California 95125 (US); ZDEBLICK, Mark, Portola Valley, California 94028 (US); FRANK, Jeremy, San Francisco, CA 94122 (US); LEI, Dino, Plano, TX 75024 (US); ADDIS, Bruce, Redwood City, California 94061 (US); COLLIOU, Olivier, Los Gatos, CA 95032 (US); SAVAGE, Georgr, M., Portola Valley, CA 94028 (US)
(74) Representative: Hylarides, Paul Jacques
(86) International application number: PCT/US2005/046811
(87) International publication number: WO 2006/069322

(56) References cited:
- EP-A- 1 356 847
- US-A- 5 515 848
- US-A- 5 515 848
- US-A- 5 571 148
- US-A1- 2003 233 134
- US-A1- 2003 233 134
- US-A1- 2005 148 832
- US-B1- 6 366 815
- US-B1- 6 473 653

## Description

### BACKGROUND

Pacing leads implanted in vessels in the body are, for many applications, flexible cylindrical devices. They are cylindrical due to three main reasons: most anatomical are cylindrical, medical sealing and access devices seal on cylindrical shapes and cylindrical leads have uniform bending moments of inertia around the long axis of the device. The cylindrical nature of the device necessitates the cylindrical design of pacing electrodes on the body of the device.

Due to the tortuous nature of the vessels in the body, following implantation the rotational orientation of one electrode can not be predetermined in many currently employed devices. As such, many currently employed lead devices employ cylindrical electrode designs that are conductive to tissue around the entirety of the diameter of the lead. This insures that some portion of the cylindrical electrode contacts excitable tissue when they are implanted. Despite the multiple devices in which cylindrical continuous ring electrodes are employed, there are disadvantages to such structures, including but not limited to: undesirable excitation of non-target tissue, e.g., which can cause unwanted side effects, increased power use, etc.

### SUMMARY

Implantable addressable segmented electrode devices, as well as methods for making and using the same, are provided. The subject devices include segmented electrode structures made up of an integrated circuit electrically coupled to two or more electrodes, where each electrode can be individually activated. Also provided are implantable devices and systems, as well as kits containing such devices and systems or components thereof, which include the segmented electrode structures.

Aspects of the invention include electrodes that are segmented, e.g., to provide better current distribution in the tissue/organ to be stimulated. In such embodiments, the segmented electrodes are able to pace and sense independently with the use of a integrated circuit (IC) in the lead, such as a multiplexing circuit, e.g., as disclosed in PCT Application No. PCT/US2005/03155 titled " Methods and Apparatus for Tissue Activation and Monitoring" and filed on September 1, 2005. The IC allows each electrode to be addressed individually, such that each may be activated individually, or in combinations with other electrodes on the medical device. In addition, they can be used to pace in new and novel combinations with the aid of the multiplexing circuits on the IC.

Aspects of the invention include embodiments in which the components are configured in a manner that minimizes mechanical stress between the components, e.g., the integrated circuit, electrodes and/or elongated conductive members. Stress minimization may be achieved in a number of different ways, e.g., by providing flexible connectors, flexible electrode designs, shaped integrated circuits, coiled conductive connectors, etc., as developed in greater detail below. Embodiments of the IC chip configurations support fatigue resistant designs for biomedical electrodes, as may be found in cardiac pacing leads or other permanently implantable or acute use devices.

In certain embodiments of the present invention, the IC chip is connected to a pacemaker with one or more, e.g., two, conductive members. The advantage of this design configuration is the reduction in the number of conductors that are required in the medical device. Prior to this invention multiple electrodes in permanently implantable leads required multiple conductors. Size and reliability have limited the number of possible discrete conductors to 2-3 max in about 9 French diameter leads with smaller diameter medical devices (e.g., 4-5 French) limited to 2 conductors.

Aspects of the invention include implantable addressable segmented electrode structures that include: an integrated circuit; and two or more electrodes coupled to the integrated circuit, wherein each of the electrodes is individually addressable. In certain embodiments, the integrated circuit is electrically coupled to at least one elongated conductive member, e.g., present in a medical carrier, where the integrated circuit may be electrically coupled to a single elongated conductive member or to two or more elongated conductive members. In certain embodiments, the integrated circuit is less than about 20mm, such as less than about 1 mm from the electrodes. In certain embodiments, the integrated circuit comprises the electrodes. In certain embodiments, the electrodes are circumferentially arranged around the integrated circuit. In certain embodiments, the electrodes are substantially aligned. In certain embodiments, the electrodes are staggered. In certain embodiments, the structure includes electrodes that are interdigitated. In certain embodiments, the structure includes electrodes of at least two different sizes. In certain embodiments, the structure includes electrodes of about the same size. In certain embodiments, the structure includes four electrodes. In certain embodiments, the structure includes three electrodes. In certain embodiments, the structure is dimensioned to fit within an implant. In certain embodiments, the structure includes is dimensioned to fit within a lead. In certain embodiments, each electrode has a surface area ranging from about 0.1 mm² to about 15 mm², e.g., from about 0.5 mm² to about 10 mm², such as about 1.3 mm². In certain embodiments, integrated circuit, electrodes and at least one elongated conductive member are electrically coupled to each other in a manner that imparts fatigue resistance to said lead assembly, where in certain embodiments at least two of the integrated circuit, electrodes and elongated conductive member are electrically coupled to each other in a manner that minimizes mechanical stress on the structure. In certain embodiments, at least two of the integrated circuit, electrodes and elongated conductive member are conductively connected to each other by a flexible conductive member. In certain embodiments, at least two of the integrated circuit, electrodes and elongated conductive member are conductively connected to each other by a liquid member. In certain embodiments, at least two of the integrated circuit, electrodes and elongated conductive member are conductively connected to each other by a coil conductive member. In certain embodiments, at least two of the integrated circuit, electrodes and elongated conductive member are conductively connected to each other by a spherical conductive member. In certain embodiments, the electrodes have a curved configuration. In certain embodiments, electrodes are flexible. In certain embodiments, electrodes comprises one or more hairpin turns. In certain embodiments, electrodes have a helical configuration. In certain embodiments, the integrated circuit comprises at least one through hole. In certain embodiments, the integrated circuit includes at least two through holes. In certain embodiments, the integrated circuit has a non-rectangular configuration, e.g., a curvilinear configuration, such as a disc-shaped. In certain embodiments, the integrated circuit is a hermetically sealed integrated circuit, e.g., that includes: an in vivo corrosion resistant integrated circuit holder having at least one feedthrough; at least one integrated circuit present in said holder; and a sealing layer; wherein the sealing layer and holder are configured to define a hermetically sealed volume in which the at least one integrated circuit is present. In certain embodiments, the structure is present in an implant or a lead, e.g., that has a circular, oval, flattened, or other shaped cross-section. In certain embodiments, the lead is a cardiac pacing lead. In certain embodiments, elongated conductive member is electrically coupled to at least one control unit, e.g., that is present in a pacemaker can.

Aspects of the invention further include implantable medical devices that include at least one implantable addressable segmented electrode structure of the invention, e.g., present in an implant or a lead, such as a cardiovascular lead, a left ventricular lead or an epicardial lead. In certain embodiments, the device is a neurological device, a muscular device, a gastrointestinal device, a skeletal device, a pulmonary device, an opthalmic device or an auditory device. In certain embodiments, the structure is electrically coupled to at least one elongated conductive member, e.g., that is electrically coupled to a control unit, e.g., that is present in a pacemaker can. In certain embodiments, the device is a cardiovascular pacing device.

Aspects of the invention further include methods of implanting an implantable medical device according to the invention into a subject; and using the addressable segmented electrode structure of the implanted medical device, e.g., to deliver electrical energy to the subject. In certain embodiments, at least a first of the electrodes is connected to a first conductive member and a second of said electrodes is connected to a second conductive member. In certain embodiments, the method includes not activating at least one of the electrodes, such as activating only one of said electrodes. In certain embodiments, the method further includes determining which of the electrodes to activate. In certain embodiments, the method further includes sequentially activating the electrodes. In certain embodiments, the method includes minimizing power consumption. In certain embodiments, the method includes activating the electrodes in manner sufficient to not stimulate the phrenic nerve. In certain embodiments, the method includes activating at least one of the electrodes of the structure to sense electrical potential in said subject. In certain embodiments, the method includes sensing conduction velocity.

Aspects of the invention further include systems and kits that include an implantable addressable segmented electrode structure according to the invention:

### BRIEF DESCRIPTION OF THE FIGURES

**FIG. 1** shows the configuration of segmented electrode structure that includes four electrodes (e.g., quadrant electrodes) positioned around an IC in an aligned configuration according to an embodiment of the invention;
**FIG. 2** provides a representation of a flexible shape for the electrodes according to an embodiment of the invention;
**FIG. 3** provides a diagram of an electrode connection to an integrated circuit that is made up of a thin flexible member, according to an embodiment of the invention;
**FIG. 4** provides a view of a medical device cross section that is not round, according to an embodiment of the invention;
**FIGS. 5A** to **5C** provide a view of a two-electrode design variation with two conductive members contacting the electrode according to an embodiment of the invention;
**FIG. 6** provides a view of a flexible connection between the back side of an integrated circuit and a form of a conductive member according to an embodiment of the invention;
**FIG. 7** shows a view of a completed assembly prior to being formed into the shape of the cross section of a medical device, according to an embodiment of the invention;
**FIG. 8** shows an integrated circuit that is bonded to the inside diameter of an electrode or electrodes according to an embodiment of the invention;
**FIGS. 9A** and **9B** show different views of an assembly according to an embodiment of the invention that provides a detail of a flexible connection from the integrated to a small diameter conductor cable, according to an embodiment of the invention;
**FIG. 10** provides a view of a medical device cross section that is not round, according to an embodiment of the invention;
**FIG. 11** provides a view of a configuration similar to FIG. 10 in a round cross section;
**FIGS. 12A** and **12B** provide different views of a medical device structure **according** to an embodiment of the invention;
**FIG. 13** provides an alternate configuration for the connection of the flexible members according to an embodiment of the invention;
**FIGS. 14A to 14F** provide details of the connection of an integrated circuit to flexible electrodes according to various embodiments of the invention;
**FIG. 15** provides a view of an alternate orientation of an integrated circuit inside a medical device assembly according to an embodiment of the invention;
**FIG. 16** provides a view of a flexible electrode assembly with a porous flexible polymeric material containing steroids, according to an embodiment of the invention;
**FIG. 17** provides a view of a final assembly that further includes a pressure sensor according to an embodiment of the invention, and **FIG. 18** provides a view of a cross-section of the assembly of **FIG. 17****;**
**FIG. 19** provides view of an electrode pattern at an angle, according to an embodiment of the invention;
**FIG. 20** provides a view of an assembly that includes two integrated circuits, according to an embodiment of the invention, where one circuit handles high power requirements and the second handles low power requirements in a medical device;
**FIG**. **21** provides a view of a shape of a flexible electrode according to an embodiment of the invention that allows the electrode to flex in two axes;
**FIGS. 22A and 22B** provide views of a lead frame which supports an integrated circuit within a quadrant electrode assembly, according to an embodiment of the invention;
**FIGS. 23A** to **23C** provide views of a simplified version of the device shown in **FIG. 22****,** where the lead and electrodes are incorporated into a single piece;
**FIG. 24** provides a view of an approach to assembly of a structure according to an embodiment of the invention;
**FIG. 25** provides a view of an approach to assembly of a structure according to an embodiment of the invention;
**FIG. 26** provides a view of an embodiment of the invention that includes a liquid electrical conductor to make the electrical connection between two electrical components, such as the chip and flexible connection to the electrode and/or the chip one or more elongated conductors;
**FIG. 27A** shows an IC that is formed into a round shape for incorporation into a medical device, according to an embodiment of the invention;
**FIG. 27B** shows a cross-section view of the IC connected to an electrode, according to an embodiment of the invention;
**FIG. 28** shows an IC connected to a multiplicity of electrodes, e.g., in a quadrant electrode configuration, according to an embodiment of the invention;
**FIG. 29** shows a coil configuration for the electrode connected to an IC, according to an embodiment of the invention;
**FIG. 30** describes an IC attached to the electrodes in a helix configuration supported by a polymer; according to an embodiment of the invention;
**FIG. 31** describes an IC connected to electrodes dispersed along the length of a medical device, according to an embodiment of the invention;
**FIG. 32A** describes an embodiment in which an IC is connected to a metallic coil by a flange on the opposite side of the chip, according to an embodiment of the invention;
**FIGS. 32B** & **C** describe views of an embodiment of the invention in which two flanges on each side of an IC are conductively connected to a conductive coil;
**FIG. 32D** describes a metallic band under the flange and coil present in certain embodiments of the invention;
**FIG. 33** describes a flange that is attached to an IC according to an embodiment of the invention;
**FIG. 34** describes an IC that is attached to electrodes with electrical cables running through the IC, according to an embodiment of the invention;
**FIG. 35** describes a formed structure of a polymer or ceramic with Pt or other suitable material formed into the structure, according to an embodiment of the invention;
**FIGS. 36A** & **36B** describe a flexible connection from the conductors to the IC, according to an embodiment of the invention;
**FIG. 37** provides a view of a mesh electrode that is attached to an IC, according to an embodiment of the invention;
**FIG. 38** provides a view of a fiber reinforced medical device with the fiber braded along the section of the device with the IC, according to an embodiment of the invention;
**FIG. 39** provides a view of an embodiment of the invention characterized by strain relief connections between the IC chip and the one or more conductors;
**FIG. 40** illustrates an overall view of the completed assembly that includes spring connectors, according to an embodiment of the invention;
**FIG. 41** illustrates a first subassembly of the embodiment shown in **FIG. 40** with the flexible connectors fitted and attached to the conductors;
**FIG. 42** illustrates a second subassembly of the embodiment shown in **FIG. 40** with quadrant electrodes molded together with PEEK;
**FIG. 43** illustrates a third subassembly which introduces the integrated circuit to the assembly of **FIG. 42****;**
**FIG. 44** illustrates a fourth subassembly of the embodiment of **FIG. 40** where the subassembly shown in **FIG. 41** is introduced into the subassembly shown in **FIG. 43****.**
**FIG. 45** provides a depiction of a cardiac resynchronization therapy system that includes one or more hermetically sealed integrated circuits coupled to lead electrodes according to an embodiment of the invention.

### DETAILED DESCRIPTION

As summarized above, aspects of the invention include implantable addressable segmented electrode devices, as well as methods for making and using the same, are provided. Embodiments of the devices include segmented electrode structures made up of an integrated circuit electrically coupled to two or more electrodes, where each electrode can be individually activated. Also provided are implantable devices and systems, as well as kits containing such devices and systems or components thereof, which include the segmented electrode structures. Embodiments of the invention are particularly suited for use in multiplex lead devices, as these embodiments can have appropriate dimensional variety of IC chips and their accompanying electrodes with internal connections, and conductive connections with structures are robust to impart fatigue resistance to the structures.

Before the present invention is described in greater detail, it is to be understood that this invention is not limited to particular embodiments described, as such may vary. It is also to be understood that the terminology used herein is for the purpose of describing particular embodiments only, and is not intended to be limiting, since the scope of the present invention will be limited only by the appended claim.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and'materials similar or equivalent to those described herein can also be used in the practice or testing of the present invention, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

In further describing aspects of the invention, aspects of implantable addressable segmented electrodes are reviewed first in greater detail, both generally and in terms of figures of certain embodiments of the invention. Next, embodiments of devices and systems, such as implantable medical devices and systems, that include the segmented electrode structures of the invention are described, as well as methods of using such devices and systems in different applications. Also provided is a description of kits that incorporate aspects of the invention.

### IMPLANTABLE ADDRESSABLE SEGMENTED ELECTRODE STRUCTURES

As summarized above, aspects of the invention include implantable addressable segmented electrode structures. Embodiments of the structures include an integrated circuit (IC) electrically coupled (so as to provide an electrical connection) to two or more electrodes. The term "integrated circuit" (IC) is used herein to refer to a tiny complex of electronic components and their connections that is produced in or on a small slice of material, i.e., chip, such as a silicon chip. In certain embodiments, the IC is a multiplexing circuit, e.g., as disclosed in PCT Application No. PCT/US2005/31559 titled " Methods and Apparatus for Tissue Activation and Monitoring" and filed on September 1, 2005. In the segmented electrode structures, the number of electrodes that is electrically coupled to the IC may vary, where in certain embodiments the number of 2 or more, e.g., 3 or more, 4 or more, etc., and in certain embodiments ranged from 2 to about 20, such as from about 3 to about 8, e.g., from about 4 to about 6. While being electrically coupled to the IC, the different electrodes of the structures are electrically isolated from each other, such that current cannot flow directly from one electrode to the other. As the structures are implantable, that may be placed into a physiological site and maintained for a period of time without substantial if any, impairment of function. As such, once implanted in or on a body, the structures do not deteriorate in terms of function, e.g., as determined by ability to activate the electrodes of the structure, for a period of at least about 2 or more days, such as at least about 1 week, at least about 4 weeks, at least about 6 months, at least about 1 year or longer, e.g., at least about 5 years or longer. As the electrodes of the subject segmented electrode structures are addressable, they can be individually activated. As such, one can activate certain of the electrodes of the structure while not activating others, e.g., in manner such that electrical stimulation can be delivered from one or more of the electrodes of the structure, but not all of the electrodes in the structure, where in certain embodiments only a single electrode of the structure is activated at any given time. As another example, one can activate one electrode in such a way that it conducts electric potentials from nearby tissue to the electric circuitry. In some embodiments, activate may further comprise electrically connecting an electrode to a conductor, such as a bus conductor, for stimulation, voltage sampling, or other purposes. In certain embodiments, the elongated conductive member is part of a multiplex lead, e.g., as described in Published PCT Application No. WO 2004/052182 and US Patent US 2004/193021.

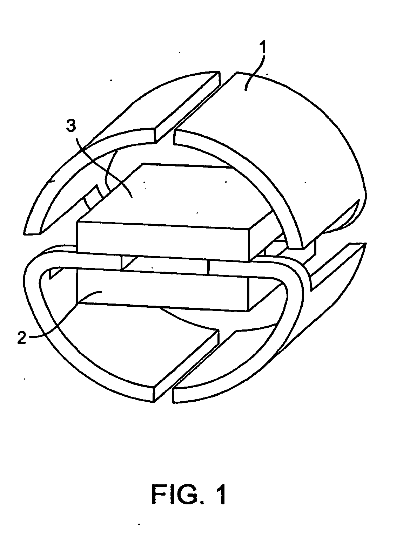

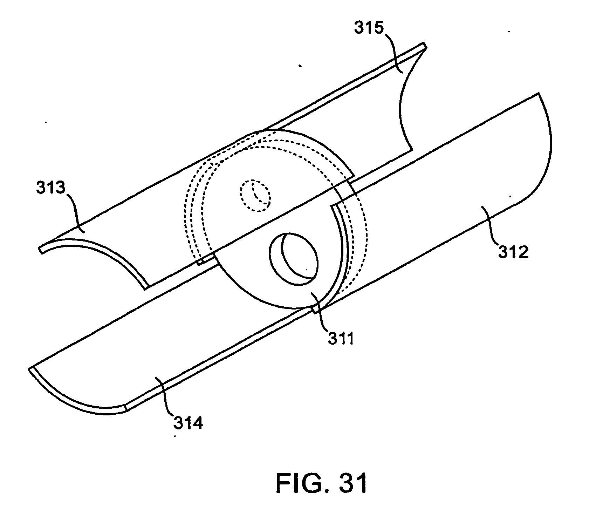

In certain embodiments, the electrodes of the segmented electrode structures are electrically isolated from each other, and may be circumferentially arranged around an IC to which they are conductively coupled. An example of such an embodiment is shown in **FIG. 1****,** where four separate electrodes are electrically coupled to a single IC in what is referred to herein as a quadrant electrode configuration. As can be seen in the figure, the electrodes are circumferentially arranged about the central IC. In the embodiment depicted in **FIG. 1****,** the segmented electrodes are arranged about the IC to form a cylinder shaped structure, which is suited for use in many different medical devices, as illustrated below. However, the structure may have any convenient shape, such as a flattened cylinder, oval shape, or other shape, as desired. In certain embodiments, the electrodes of the segmented electrodes are aligned, e.g., having one edge, e.g., the proximal edge, of each electrode shares a common plane as shown in **FIG. 1****.** In yet other embodiments, the different electrodes may be present in an offset configuration, for example in a staggered configuration, e.g., as shown in **FIG. 31****.** By "staggered" is meant that at least one of the edges of the electrodes does not share a common plane. In yet other embodiments, the electrodes may have an interdigitated arrangement.

In embodiments of the invention, the structures are dimensioned to be placed inside a lead, e.g., cardiovascular lead, epicardial lead, left ventricular lead, etc., or implant. By "dimensioned to be placed inside of a lead or implant" is meant that the structures have a sufficiently small size (i.e., form factor) such that they can be positioned inside of a lead or implant. In certain embodiments, the hermetically sealed structures have a longest dimension, e.g., length, width or height, ranging from about 0.05 mm to about 20 mm, such as from about 0.2 mm to about 5 mm, including from about 0.5 mm to about 2 mm. Accordingly, embodiments of the structures allow the practical development of miniaturized, implantable medical devices for days, months, and even years of practical, reliable use.

In certain embodiments, the segmented electrode structures are electrically coupled to at least one elongated conductor, which elongated conductor may or may not be present in a lead, and may or may not in turn be electrically coupled to a control unit, e.g., that is present in a pacemaker can. In such embodiments, the combination of segmented electrode structure and elongated conductor may be referred to as a lead assembly.

Embodiments of the invention include implantable fatigue resistant structures. In such embodiments, at least the IC and electrode components of the segmented structure, for example, the IC, electrode and conductor components of a lead assembly, are electrically coupled to each other in a manner that imparts fatigue resistance to structure and/or lead assembly that contains the structure. This fatigue resistance ensures that the structures can survive intact (i.e., without substantial, if any, breakage of the connections between the integrated circuit and electrode(s) components of the structure) in an in vivo environment, such as in a physiological environment in which they are in contact with blood, and/or tissue. Because the structures are implantable, the implantable structures are structures that may be positioned in or on a body and function without significant, if any, deterioration (e.g., in the form of breakage of connections, such as determined by function of the segmented electrode structure) for extended periods of time. As such, once implanted, the structures do not deteriorate in terms of function, e.g., as determined by function of an integrated circuit and electrodes coupled thereto of the structure, for a period of at least about 2 or more days, such as at least about 1 week, at least about 4 weeks, at least about 6 months, at least about 1 year or longer, e.g., at least about 5 years or longer.

Aspects of the invention include one or more features that impart fatigue resistance to the subject segmented electrode structures. Fatigue resistance imparting features include, but are not limited to: electrical connections between components, e.g., electrodes, IC, elongated conductive members, that minimize mechanical stress between the connected components. For example, flexible conductive connectors of a variety of different materials and/or configurations are employed in certain embodiments of the invention, as described in greater detail below. In yet other embodiments, liquid conductive connectors of a variety of different materials and/or configurations are employed which provide for a high degree of freedom of movement between connected components, as described in greater detail below. In yet other embodiments, non-bound conductive connectors of a variety of different materials and/or configurations, e.g., rigid spheres, coils/springs, etc., are employed which provide for a high degree of freedom of movement between connected components, as described in greater detail below. In these embodiments, "non-bound" means that the connector is not physically immobilized on a region of the connected component, but is instead capable of moving across a surface of the connected component, at least in some plane, while still maintaining the conductive connection.

In certain embodiments, the IC component of the structures is hermetically sealed, e.g., it is present in a hermetically sealed structure that includes a hermetically sealed volume which houses one or more ICs. Aspects of the invention include hermetically sealed ICs that include: an in vivo corrosion resistant holder having at least one conductive feedthrough; and a sealing layer; where the sealing layer and the holder are configured to define a hermetically sealed volume, e.g., in which one or more ICs is present. Such hermetically sealed structures are further described in copending PCT patent application serial no. PCT/US 2005/04681 titled "Implantable Hermetically Sealed Structures," and filed on even date herewith.

The advantages of the present innovation of separately addressable segmented, e.g., quadrant electrodes, are many fold. Because the distribution of electrical potential (e.g., cardiac pacing pulse) can be directed, a great flexibility is provided in clinical applications. For example, by selectively activating one or more of the electrodes of the segmented structure, electrical current can be directed to only that tissue that needs to be excited, thereby avoiding excitation of tissue that is not desired to be excited. This feature provides multiple benefits. For example, in prior art methods, a left ventricular pacing electrode would typically have to be disabled, and the cardiac resynchronization therapy (CRT) intervention terminated, if phrenic nerve capture by the electrode caused the patient to suffer a diaphragmatic spasm with each discharge. By the careful electrode selection to control the directionality of electric current provided by the present invention, capture of the phrenic nerve can often be avoided, while appropriate levels of cardiac stimulation are maintained.

In addition, any given electrode can have a small surface area and still adequately excite the tissue that needs to be excited. For example, electrodes having a surface areas ranging from about 0.1 mm² to about 4.0 mm², such as from about 0.5 mm² to about 3.0 mm² may be employed. Despite their small surface area, excitation of that tissue that needs to be excited is achieved. When the segments are distributed around the circumference of a pacing lead, excitable tissue will be contacted regardless of the rotational orientation of the device in the vessel. With the reduced surface area of the electrode segments, the impedance is larger than that of a ring electrode of equal axial length thereby reducing the current drain on the pacemaker; which can lead to improved longevity of the device. Experimental data from epicardial left ventricular pacing with a four segment electrode structure have demonstrated an eight-fold difference in capture threshold between those segments that are in contact with cardiac tissue and those which are not. As such, with appropriate segmented electrode configuration, capture threshold differences of ten-fold or more may be achieved. The capture threshold, as defined as the minimum voltage that initiates excitation of the heart tissue, is directly proportional to power consumption of a pacemaker.

The inventive use of separately addressable quadrants on a multiple electrode leads allows a number of other clinical advantages. In many cases, the present invention allows patients who would be non-responsive using prior art devices to become responsive to treatment. For example, multiple potential excitation positions along the lead allows for selection in real time of the most advantageous pacing, without requiring repositioning of the lead. Synergistic use of multiple points of stimulation are also available (simultaneously or sequentially), again without any further lead positioning. Currently available techniques require difficult and often unsuccessful repositioning of the lead when an effective excitation position is not achieved. Because of difficulties in variations of anatomical features, and limitations in time available for repositioning, often results are sub-optimal or poor. Additional advantages include the ability to achieve fine measurement of conduction velocity in different axes.

In addition, in electrical tomography embodiments such as those described in U.S. Provisional Patent Application No. 60/705,900 titled "Electrical Tomography" filed 8/5/05, the subject structures permit calibration of local electric field gradients to improve accuracy in synchrony quantification and possibly enable absolute measurements (e.g., stroke volume, ejection fraction, etc.). In electrical tomography applications, applied electric fields are distributed in a curvilinear fashion within the body. Knowing the local field gradient in the region of interest (e.g., a cardiac vein overlying the LV) permits absolute determination of the local relationship between electrical distance (gradient) and physical distance.

Embodiments of the segmented electrode structures may include one or more of the above features, or others. In further describing the invention, embodiments of the structures are now reviewed in greater detail in terms of the figures.

As mentioned above, FIG.1 provides a representation of a segmented electrode structure according to an embodiment of the invention. Cardiac pacing electrodes of the present invention may vary, and in certain embodiments range from about 0.1 to about 4 mm² in area, e.g., about 1.5mm² in area. The electrodes can be positioned relative to the IC in a variety of different formats, e.g., circumferentially around the IC and/or the body of a lead, or they could be distributed longitudinally along the length of the lead body, extending from the connection from the IC or they could be arranged in a pattern that improves tissue contact or that facilitates measurement of local electrical field gradients.

A configuration of electrodes around the IC according to an embodiment of the invention, which is referred to herein as a quadrant electrode embodiment, is shown in FIG. 1. The four electrodes 1 are distributed around the IC in a circumferential pattern. Electrode 1 is shown as a solid surface but it may have a finer scale pattern formed into the surface that improves the flexibility of the electrode. IC chip 2 is hermetically sealed and provides a multiplexed connection to conductors in the lead (not shown in this figure). Optionally, top cap 3 is bonded to the integrated circuit. Cap **3** is a component that helps support the electrode to integrated circuit connection. Cap **3** may contain additional circuits or sensors. In certain embodiments, this assembly is incorporated into a flexible material, e.g., polymeric material, to form the body of the device. The device may be round or some other shape best suited to the particular location in the body where it is intended to be deployed.

The materials of construction of the conductive members, e.g., electrodes, for use with the presently described ICs may be primarily platinum, or platinum alloy, including platinum 5% iridium, platinum 10% iridium, or platinum 20% iridium. Additional appropriate platinum alloys include, but are not limited to: platinum 8% tungsten, platinum nickel, and platinum rhodium. The alloy could also be gold tin with gold 20% tin alloy. An additional material for the electrode of the present invention can be titanium. The titanium could be plated with platinum or platinum alloys previously described. Corrosion resistant alloys can also be deposited by RF Sputtering, electron beam vapor deposition, cathodic arc deposition, or chemical vapor deposition, among other methods. In addition to titanium, base electrode materials can include stainless steel, e.g., 316SS, or cobalt based super alloys, e.g., MP35N, or tantalum. The electrode can.also be electroformed.

The electrodes may be fabricated from bulk cold-worked alloys. In addition the electrodes can be formed wholly from thin film deposition processes. The electrodes formed from bulk metal or alloys can take advantage of a fine microstructure formed by cold working to the final thickness. Refined microstructures typically increase the yield point of the material and the fatigue life of the material.

Electrodes formed by thin film processes can be made with the same class of materials described previously. The electrodes can also be fabricated as layered structures that exploit different material characteristics for optimum performance. High strength metals or alloys can be deposited for optimum strength as base layer. Additional corrosion resistant layers could be formed above the base layers. The final coatings could be materials that enhance the electrodes ability to transfer charge to tissue or to sense electrical signals. In addition other coatings on the electrode could enable chemical sensing, pH measurements, pressure measurement, or ultrasound detection.

The fabrication process from bulk metals or alloys can be done by any convenient method, such as methods employed for fabrication of cardiovascular stents and other passive mechanical devices. The electrode can be manufactured by laser cutting, Electric Discharge Machining (EDM), photochemical etching, or by stamping and forming or a combinations of those fabrication processes. In addition, that electrode can be chemical etched, or electro-polished to produce a smooth surface. Smooth surfaces are desirable for fatigue resistant devices to reduce the number of potential crack initiation sites.

Additionally the electrode can be formed by vacuum deposition of a suitable metal or alloy on a fabric or a polymeric film that would cover the outside surface of the medical device. The sputtered area can then be plated to additional thickness if required. This allows the fabric to perform two functions-first to reinforce the lead from applied mechanical effects and second to provide a flexible substrate for a conductive electrode. This configuration reduces the abrupt change in bending stiffness that results from a change in materials along the length. This conductive area is then connected to the IC chip with a flexible conductive member.

In the present invention, the surface of the conductive member(s), e.g., electrodes, may be different than the bulk material. The surface that is exposed to the blood stream should survive corrosion and electrolytic corrosion that occurs in that environment. In addition the surface should maximize the charge transfer to the tissue for pacing. The surface, in certain embodiment, will optimize sensing of electrical signals. The surface can also provide the ability to sense chemical species or pH changes.

The surface coating may include elements of in the noble metal family including the alloys; oxides and nitrides (platinum, platinum iridium, titanium nitride, and iridium oxide). In addition these materials can increase the micro roughness of the electrode, increasing the microscopic surface area. This improves the capacitive charge transfer ability of the electrode.

Additionally coatings can be applied to the inventive structures to reduce electrolytic corrosion of electrodes when paced outside of the water window. Electrodes in saline solutions can experience various degradation mechanisms when electrically driven at voltages about below -0.6 V or about above 0.8V. These voltages define the water window where outside these ranges water decomposes to H+ or OH-. When these ions are produced they raise or lower the pH. pH changes can cause degradation of the electrode material or material that is in close proximity to the electrode.

pH changes can also cause degradation of tissue when used for electrical pacing. At sufficiently high voltages, Cl- ions can be produced in saline solutions. These ions can form corrosive chemical species. Another degradation mechanism is caused by the production of H+ in alternating current applications. The H+ can be driven back and forth through thin film electrodes causing mechanical destruction of the electrode. This has been observed on thin film Pt electrodes. For electrodes made from Pt and Pt group metals the production of destructive ionic species is increased due to the catalytic nature of Pt. This reduces the usefulness of a material that is very stable in saline solutions due to its nobility.

It is known that S, Ca and select other elements and compounds can "dope" the Pt group metals used in catalytic converters. This is normally considered a detrimental effect. For the use of Pt group materials as electrodes doping to produce a change in the function of the Pt group can be innovatively useful. A doped electrode would continue to retain its noble metal properties of chemical resistance but with the small additions of S or select other element the catalytic nature of the Pt can be reduced.

The innovation of providing doping of the surface or body of the electrode in this manner reduces the generation of H+, OH- and Cl- ions in saline solutions. The reduction of these ionic species reduces the changes in pH in saline solutions near the electrode and the destructive effects of those pH excursions.

The S, Ca or other doping elements can be introduced at the ppm level during the deposition of thin film Pt electrodes. They can also be incorporated into the base alloy during melting for the fabrication of thicker electrodes. They can also be deposited onto the surface by emersion into a fluid.

Embodiments of the invention include the use of flexible conductive connectors between different components. The conductive connectors of these embodiments are flexible in that allow a degree of movement of in least one axis of rotation without breaking. As such, one of the components may move relative to another without the stress being transmitted to the other component, such that the other component does not move. Furthermore, movement of one component does not result in breakage of the conductive connection with the other component. Flexible conductive connections may be provided with a number of different connection configurations, including but not limited to: bonded solid connections, e.g., made of flexible materials; non-bonded solid connections, e.g., ball bearing connections, spring connections; fluid connections, etc.

As reviewed above, embodiments of the invention further include the use of electrode configurations, e.g., that impart flexibility to the electrodes minimize mechanical stress between the electrode and the integrated circuit. Electrode design configurations of interest include, but are not limited to: curved electrodes, bent electrodes, segmented electrodes, helical electrodes, and the like.

Embodiments of the invention further include the use of shaped ICs, where these shaped-chips have a non-rectangular configuration, e.g., a curvilinear configuration, such as a disc configuration. Aspects of these embodiments include the presence of one or more holes in the middle of the chips, e.g., which provide through ways for conductive members. Aspects of these embodiments further include electrodes that are directly bonded to the edges of the chips.

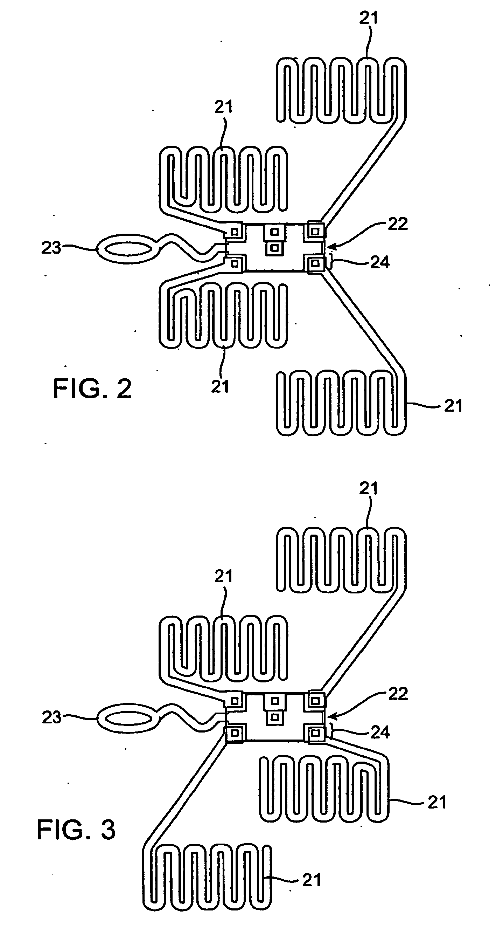

One representation of the flexible shape for the electrodes is shown in **FIG. 2** where the electrodes **21** have a bent configuration, e.g., made up of multiple hairpin turns. Such flexible hairpin turn containing electrodes may be fabricated a number of different ways. For example, the electrodes of these embodiments may be produced by having slots cut in the parent electrode either through laser cutting, EDMing or chemically etching the shape. In addition a thin film electrode could have this shape defined by photolithography, with the electrode then being built by plating or vapor deposition process. One advantage of this shape of electrode is that it reduces the bending stresses (EI) of this stiff member. Greater flexibility of the electrode in turn reduces the stresses that are applied to the IC inside the medical device. It also allows the overall device to become more flexible by reducing the trauma to tissue caused by repeated contact with a hard device.

An additional advantage of this inventive design over previous designs is that the flexible electrode can provide paths through the electrode so that pharmacological agents (i.e., drugs) which may be positioned, e.g., in a delivery vehicle, such as a depot, under the electrodes, e.g., so that the pharmacological agent can leach out.

Agents that may be present in a drug delivery vehicle, e.g., depot, associated with the electrode include, but are not limited to: Therapeutic agents may be, for example, nonionic or they may be anionic and/or cationic in nature. Exemplary non-genetic therapeutic agents for use in connection with the present invention include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular-cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (I) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines, and (r) hormones. Of interest in certain embodiments are anti-inflammatory agents, e.g., glucocorticosteroids, such as dexamethasone, etc.

In certain embodiments, a pharmacological agent is present in a polymeric matrix that is proximal with the electrodes, e.g., positioned under the electrodes in a polymer matrix; or over the electrodes in a polymer matrix. In certain of these embodiments, pharmacological agents of interest are anti-thrombotic agents such as heparin, heparin derivatives, urokinase, and PPack (dextrophenylalanine proline arginine chloromethylketone). In certain embodiments, pharmacological agents of interest are anti-inflammatory agents such as dexamethasone, prednisolone, corticosterone, budesonide, estrogen, sulfasalazine and mesalamine. In certain embodiments, pharmacological agents of interest are antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors. In certain embodiments, pharmacological agents of interest anesthetic agents such as lidocaine, bupivacaine and ropivacaine. In certain embodiments, pharmacological agents of interest are anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides. In certain embodiments, pharmacological agents of interest are vascular cell growth promoters such as growth factors, transcriptional activators, and translational promotors. In certain embodiments, pharmacological agents of interest are vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin. In certain embodiments, pharmacological agents of interest are protein kinase and tyrosine kinase inhibitors (e.g., tyrphostins, genistein, quinoxalines). In certain embodiments, pharmacological agents of interest prostacyclin analogs. In certain embodiments, pharmacological agents of interest cholesterol-lowering agents. In certain embodiments, pharmacological agents of interest angiopoietins. In certain embodiments, pharmacological agents of interest are antimicrobial agents such as triclosan, cephalosporins, aminoglycosides and nitrofurantoin. In certain embodiments, pharmacological agents of interest are cytotoxic agents, cytostatic agents and cell proliferation affectors. In certain embodiments, pharmacological agents of interest are vasodilating agents. In certain embodiments, pharmacological agents of interest are agents that interfere with endogenous vasoactive mechanisms. In certain embodiments, pharmacological agents of interest are inhibitors of leukocyte recruitment, such as monoclonal antibodies. In certain embodiments, pharmacological agents of interest are cytokines. In certain embodiments, pharmacological agents of interest are hormones. In certain embodiments, pharmacological agents of interest are anti-inflammatory agents, e.g., glucocorticosteroids, such:as dexamethasone, etc.

The agent may be present in any convenient delivery vehicle, e.g., one that can be positioned in the structure, e.g., proximal to one or more of the electrodes thereof. Structures of interest include, but are not limited to: the drug delivery structures disclosed in U.S. Patent No. 4,506,680, the disclosure of which delivery structures is herein incorporated by reference.

Steroids are used to reduces pacing thresholds. The electrode configuration provided in **FIG.2** provides for the ability to place steroids at the exact electrode location, which is not possible with solid electrodes. The steroids may be present in any convenient depot composition. In **FIG. 2****,** shaped (e.g., hairpin turn containing) electrodes **21** are bonded to IC **22** via connections **24.** Also shown is flexible connection **23** with provides a flexible conductive connection to an elongated conductive member.

**FIG. 3** shows a staggered arrangement of electrodes **21** wrapped around the circumference of the IC **22** and flexible connection **23** for electrically coupling to an elongated conductor. While **FIG. 3** shows four electrodes, there can be a multiplicity of electrodes anywhere from one to four or greater number of electrodes, the only limitation being the number of connections available on the chip or satellite. The size of the electrodes may vary, and in certain embodiments ranges on the order of about 1.5 mm² squared but could range from about 0.1mm² to about 4 mm². This size determination is generally based on the anticipated clinical usage of the particular electrode and its position on the medical device. The electrode connections of the electrodes to the chip are thin flexible members to reduce the amount of stress that is applied to the chip, as shown in **FIG. 3****.** In certain embodiments, the thin connectors have a longest cross-sectional dimension ranging from about 0.025 mm to about 2.5 mm, such as from about .075 mm to about 0.25 mm.. By flexible is meant that the connectors may be bent at least quarter of the way around the circumference of a rod having a diameter of 4 mm without breaking. The figure shows straight elements but the form of the elements may include bends and curves to avoid the conductors in the medical device. The bends and curves can also improve the fatigue life of the device by reducing the stress on the member by increasing the strain that can be accommodated without causing plastic deformation or crack propagation. The figure shows the device assembly prior to being formed into a cylinder or other shape to match the medical device cross section in which the structure is to be positioned.

The flexible connection members may have a multiplicity of configurations into which they can be formed as they extend off the IC chip. These designs can be both for a bulk electrode design with an electrode that has a material thickness of about 75µm or a thin film electrode with a conductor thickness of about 10µm to about 300µm. The electrodes may also have a polymeric support of poylimide (thin film process) or PEEK (thermoformed). The polymeric material may also have openings cut or formed into it to increase the medical devices flexibility.The two main inventive designs for the electrodes are either a bulk material or a thin film material. The bulk material version would typically have a material thickness of about 75 µm but that thickness could range from about 10 µm to about 300 µm depending on the particular requirements. The thin film version of the electrode could have a thickness of about 0.1 µm to about 100 µm depending on the particular production methods and the design requirements.

The connection between the inventive electrode and the IC can be made with conductive polymeric materials, where a polymeric material is loaded with a material that would be conductive, where the conductive filler or doping agent may have a variety of different configurations, e.g., spheres, rods, ingots, or irregular shapes, and made from a variety of different materials, e.g., metals, both pure and alloys, carbon, etc., where specific conductive materials of interest include, but are not limited to: nickel spheres, e.g., having a size range of about 5 µm that are coated with gold, silver, or platinum, carbon fibers or carbon nanotubes, etc.

The inventive electrodes can be connected to the IC with a suitable solder, such as a noble metal solder, Pt-Sn, Pt-Ge, or Au-Sn where gold 20% tin and gold silicon are two examples of a suitable solder that would provide a conductive connection between the electrodes and the chip. This joining method covers a wide surface area of the IC chip. Advantages of this design include a large surface area helps distribute stresses throughout the chip and additional hermetic sealing for the electronics under the area of connection. In certain embodiments, the solder and electrodes that are connected in the area of connection have similar electrochemical characteristics to reduce corrosion, e.g., galvanically induced corrosion. In addition, attachment methods of the present invention can include wire bonding and riveting and chip bonding where the electrodes and chips are encapsulated to an assembly. These attachment methods can be performed both on the thin film design version and the bulk electrode design version. In certain embodiments, the connections of the chip interface are wide, e.g., at least about 0.25 mm wide, such as at least about 1.25 mm wide, to distribute stresses over a larger area. There can be a multiplicity of conductors to each electrode to provide redundancy.

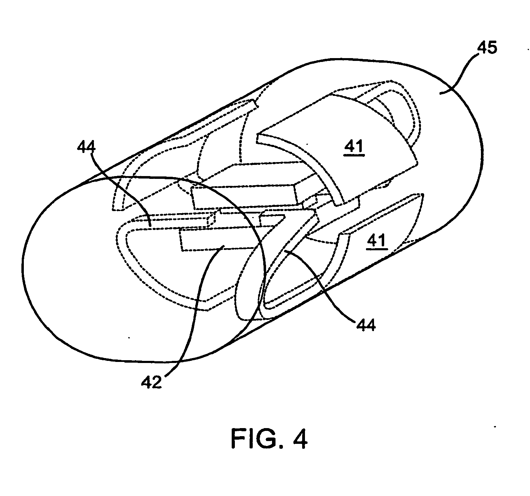

An additional configuration of flexible members **44** connecting curved planar electrodes **41** to an IC **42** is shown in **FIG. 4****.** The stress applied to the IC is reduced by increasing the amount of elastic strain the member can withstand, e.g., using materials and/or configurations as described above. In **FIG:4****,** the fatigue resistant IC/electrode structure is present in a lead body **45,** and the outer curved surface of the electrodes **41** matches the configuration of the lead body.

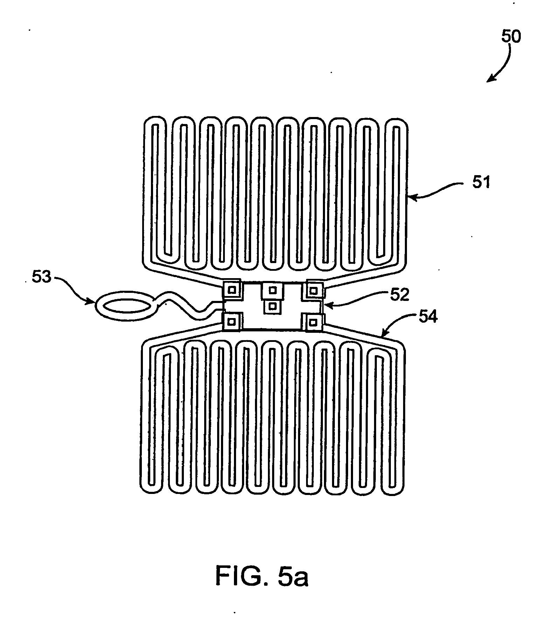

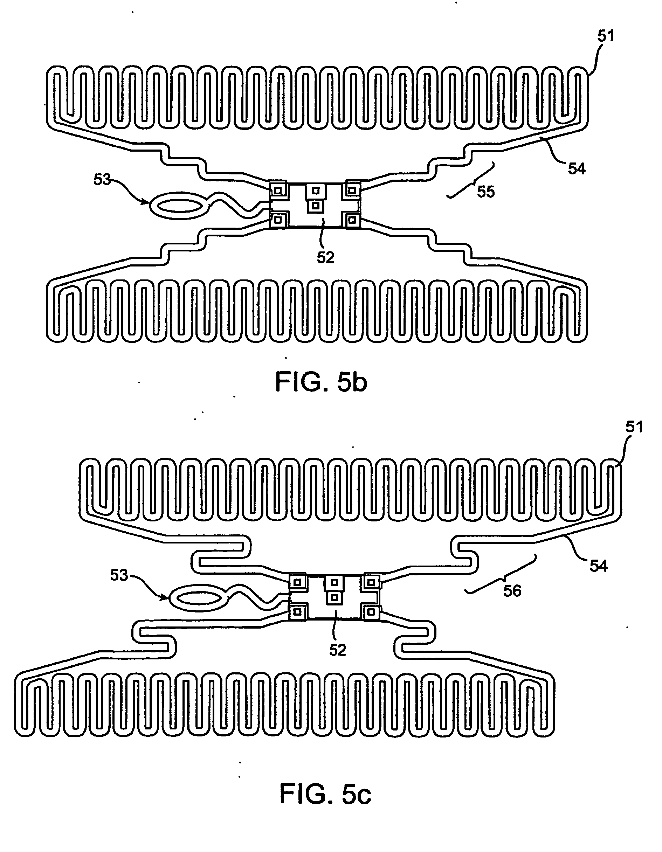

**FIG. 5A** shows a two-electrode design variation of the IC chip/electrode structure **50** with two conductive members **54** contacting each electrode **51.** Also present is flexible connection **53** for conductively coupling the structure, e.g., to an elongated conductor. Flexible conductive member **54** connects the electrodes **51** to the IC chip **52.** **FIG. 5B** shows a two conductor **54** electrode **51** design with bends **55** formed into the conductive member **54.** The bends **55** serve to increase the flexibility of the member. **FIG. 5C** shows a variation of the curve pattern or bends **56** to provide flexibility in a defined direction. Other variations for curves or shapes of the conductive members **54** can be employed, such as those employed in the art of stent design.

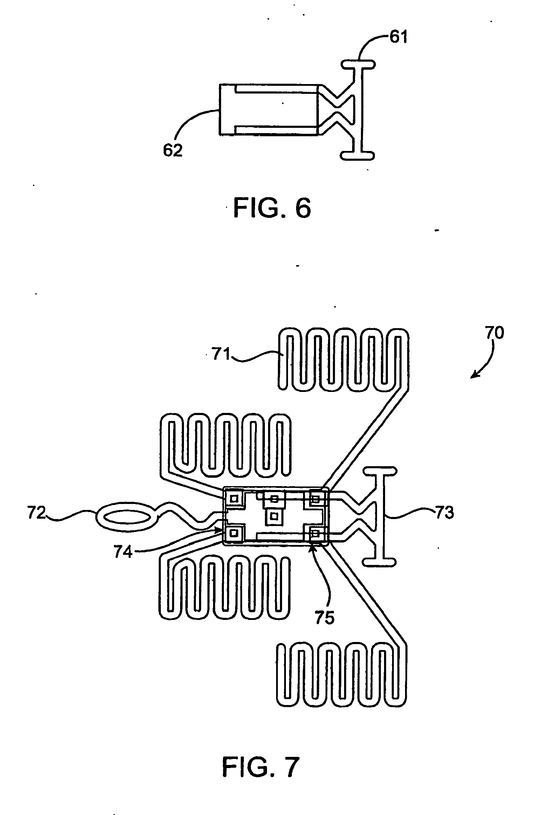

**FIG. 6** shows a flexible connection between the back side of an IC chip and a form that is soldered welded or crimped onto a conductive member. Metallic form **61** extends from one side .only of the assembly to reduce the possibility of tensile stresses being transmitted through the assembly. The form is metallurgically bonded onto the bottom of the IC **62.** **FIG. 7** shows a completed assembly **70** prior to being formed into the shape of the cross section of the medical device. Shown are the flexible electrodes **71,** flexible connection **72** to a conductive member, and flexible connection **73** to another conductive member, such as a conductive multi-filar coil with a diameter of about .25 to 1.25 mm, such as from about .5 to about **1** mm and filar diameters of about 0.01 to about 0.1, such as from about 0.05 to about 0.1 mm. IC **74** allows multiplexed connections to the electrodes. Polymeric material **75** is insert molded or thermoformed over the IC to electrode connections, PEEK, PEKK, Ultem or FEP.

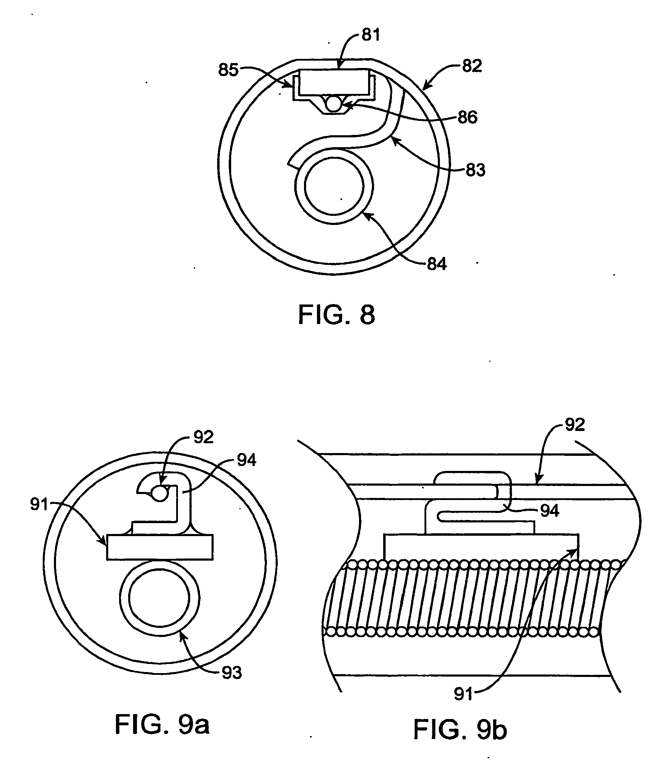

**FIG. 8** shows an IC **81** that is bonded to the inside diameter of an electrode or electrodes **82.** The connection may be of any type, e.g., a gold tin solder joint or other method, e.g., as reviewed above. Segmented electrodes **82** are electrically coupled to IC. Flexible conductive member **83** is connected to a multi-filar coil **84,** e.g., as described for **FIG. 7****,** where this multi-filar coil comprises an elongated conductive member, e.g., for providing a conductive connection to a control unit, e.g., present in a pacemaker can. Also shown is material **85** which partially or fully encapsulates the IC chip **81,** e.g., to provide a hermetic seal to the chip, where the material may be polymeric or other type of encapsulating material. Second conductive member **86** is joined to the IC Chip.

**FIGS. 9A** and **9B** show a detail of a flexible connection **94** from the IC chip **91** to a small diameter conductor cable **92,** e.g., present as a stranded cable. Also show is a multi-filar cable **93.** The connection to the IC chip is not shown in these two figures. **FIG. 9B** shows a cross section view of the flexible conductive member connecting the IC to the conductive cable **92.** These flexible conductive members **94** can be fabricated in similar manner to the fabrication methods described earlier for the electrodes. The electrodes are not shown in these figures.

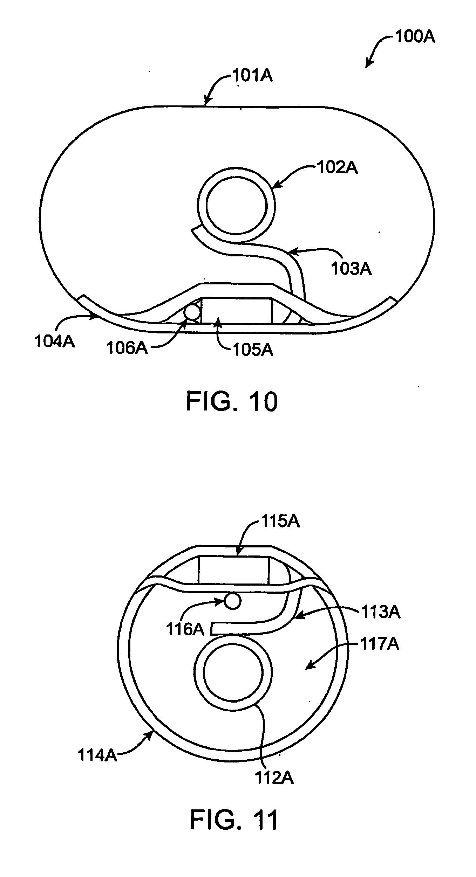

**FIG. 10** shows a medical device cross section **100A** according to an embodiment of the invention, where the medical device is not round. The electrodes are distributed on one or more of the longer axes. This inventive configuration is designed to navigate and be implanted in spaces between two or more organs, e.g., the epicardium and the pericardial sack for cardiac application. This configuration could also be utilized for pacing and sensing the stomach. Medical device body **101A** may be fabricated from any convenient material, e.g., extruded silicone or urethane. Multi-filar coil conductor **102A** is as described above. The coil provides passage of a guidewire or stylet to guide the device when it is implanted. Flexible conductive member **103A** connects the coil **102A** to the electrode **104A** or IC chip **105A.** In this figure the electrode **104A** has a portion formed in such a manner to capture the IC chip **105A.** Second conductor **106A** is typically a stranded conductive cable, e.g., as described above. This design can be used with a multiplexed IC chip or alternately this design can be used with hard wired configuration with one or more electrodes.

**FIG. 11** shows a configuration similar to **FIG. 10** in a round cross section. Medical device body **111A** may be fabricated from any convenient material, e.g., extruded silicone or urethane. Multi-filar coil conductor **112A** has been described earlier. The coil **112A** provides passage of a guidewire or stylet to guide the device when it is implanted. Flexible conductive member **113A** connects the coil to the electrode **114A** or IC chip **115A.** In this figure, electrode **114A** has a portion formed in such a manner to capture the IC **115A.** Second conductor **116A** may be a stranded conductive cable, e.g., as described above. This design can be used with a multiplexed IC chip or alternately this design can be used with hard wired configuration with one or more electrodes.

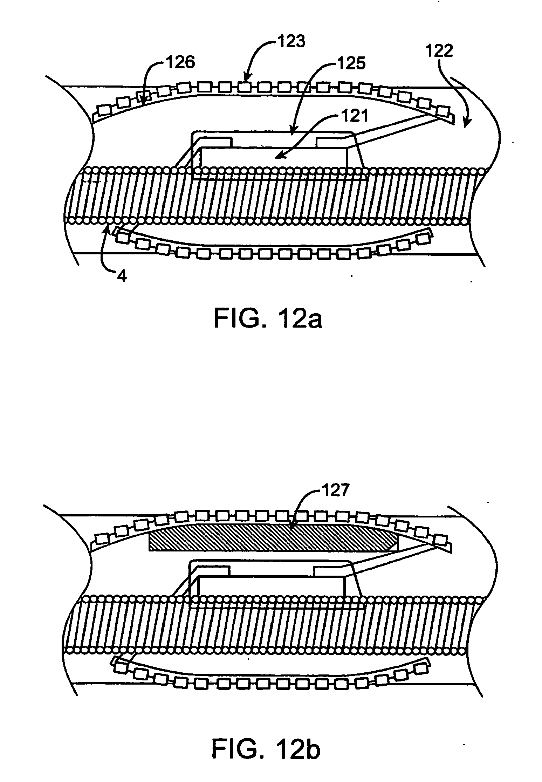

**Fig. 12A** shows a cross section of the medical device **120** according to an embodiment of the invention. Shown are the IC chip **121** and the medical device body **122** described earlier. Electrode **123** is formed into a flexible pattern as described earlier, e.g., see **FIGS 5A to 5C****.** Also present is multi-filar cable conductor **124.** Item **125** is an encapsulating material, e.g., a polymeric material such as PEEK, PEKK or FEP, that is insert molded or thermoformed over the electrode to chip and conductor connection elements. Polymeric material **126** is e.g., PEEK, PEKK or FEP molded on to the flexible electrode. Additionally polymeric material **126** is formed with cuts or holes to provide flexibility. Holes and cuts in polymeric material **126** provide locations for the polymeric body of the device to flow during molding. This provides additional structural integrity to the medical device. The electrode is formed into the shape of the medical device cross section. The electrode is additionally formed or molded such that the ends are inside the body of the medical device. This detail of design provides among other benefits, a reduction in stresses arising from bending because the diameter of the stiff elements **123** and **126** determines the bending stress. An additional advantage with a flexible design for the electrodes is to distribute stresses and taper stresses at the edge of the IC chip. The electrodes are placed so that they overlap the edge of the IC chip inside the medical device. This configuration provides smoother transitions in the bending stresses along the length of the design, which enhances long term fatigue survivability when implanted in the body. This inventive embodiment configuration also aids in the removal of the medical device after implantation. These medical devices can be implanted for 10 years or more. Within 3-6 months of implantation a stiff tissue capsule forms around the device. If the device fails or needs to be removed current practice is to tunnel down the device with a hollow cutting catheter. Sharp edges or discontinuities cause the edge of the cutting catheter to hang up. The failure of the cutting catheter to extract the lead requires the patient to have the medical device extracted in an open surgical procedure. **FIG. 12B** shows an area **127** under the electrode that contains steroid, such as dexamethasone, in a depot composition, e.g., present in an initial dosage of from about 0.5 to 1.0 mg. The pharmacological agent, e.g., steroid, is incorporated into the depot, e.g., a flexible polymeric material, when the medical device is assembled. Alternately the steroid is impregnated into a porous polymeric material, such as PTFE or an open cell foam. The steroid can leach out at the electrode site to reduce pacing thresholds.

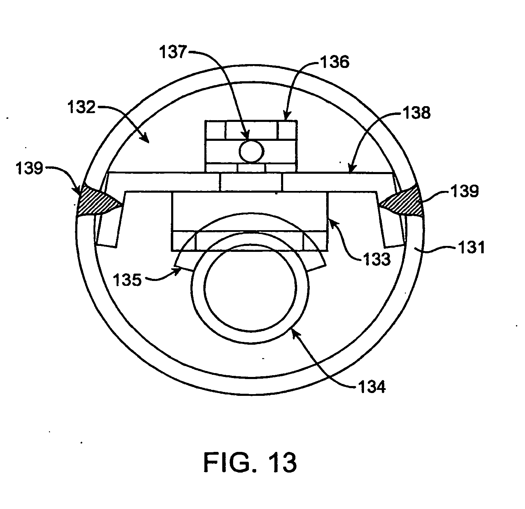

**FIG. 13** shows an alternate configuration for the connection of the flexible members **138** to the flexible electrodes **131.** The flexible members **138** are joined to the IC chip **133,** e.g., as described above. The members are formed to aid the metallurgical connection with the electrode. They can be joined by welding, laser welding or soldering, e.g., with a noble metal solder. A weld zone **139** is formed between flexible members **138** and flexible electrodes **133.** Body of the medical device **132** is as described above. Flexible conductive member **135** joins the IC chip item **133** to conductive multi-filar conductor coil **134.** Flexible conductive member **136** connects the stranded conductor cable **137** to the IC chip **133.**

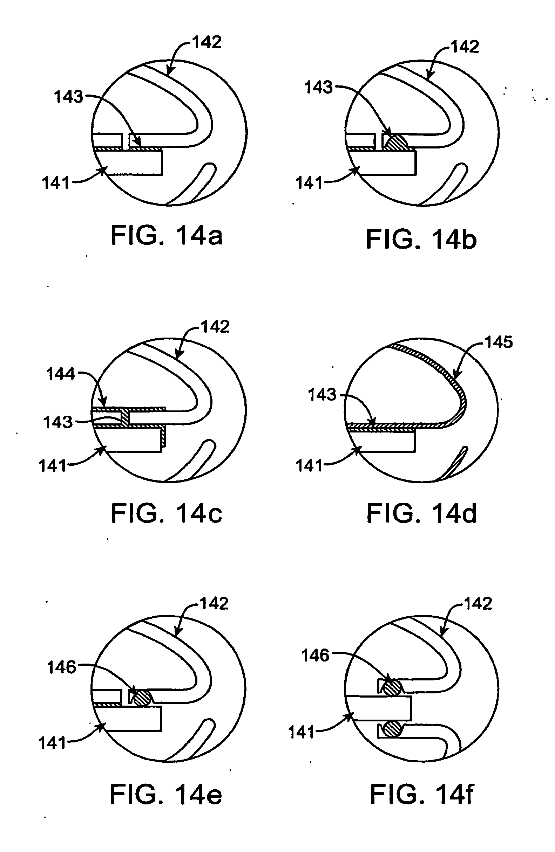

**FIGS. 14A** to **14F** provide representations of various different types of connections that may be made between the electrodes and the IC chip. **FIG. 14A** shows a detail of the connection of IC chip **141** to the flexible electrode **142.** Noble metal solder **143,** Pt-Sn, Pt-Ge can be gold 20% tin, gold silicon, or pure gold. The attachment is done at the melting temperature of the solder or at a lower temperature when sonic energy and/or pressure is additionally applied. The metallurgical bonding is done on the flat patterned electrode and then the device is formed into the cross sectional shape of the medical device.

**FIG. 14B** shows a detail of the connection of IC chip **141** to flexible electrode **142.** Conductive polymeric material **143** is typically silicone filled with conductive materials. The conductive materials may be as described above, e.g., Ni balls 5µm in diameter with silver, gold or Pt coatings. Additionally the silicone can contain carbon fiber or carbon nanotubes. The conductive material can also be ferro-fluids or conductive gels or fluids. The electrode can provide a pocket or chemically etched space to contain the flexible conductive material. This configuration allows electrical connection between the IC chip and electrode that does not transmit mechanical forces, e.g., tensile forces, to the bonding pad at the surface of the IC chip.

**FIG. 14C** shows the configuration described in **FIG. 14A****.** In addition the electrode to pad joints are supported by a polymeric material **144.** This material serves to support the assembly and reduce forces that are transmitted to the bonding pad / electrode connection. This item can be applied to a number of contact variations. The polymeric material can be PEEK, Ultem or FEP. These particular polymers can be thermoformed or insert molded to fill the small gaps in the assembly. These materials have been shown to be biocompatible and stable for implant applications.

**Fig. 14D** shows a thin film flexible electrode **145** bonded to the IC chip **141** by gold tin solder **143** or conductive polymers. **FIG. 14E** shows contact with the IC chip **141** with the electrode **142** by way of a spherical conductive member **146.** The sphere may be fabricated from any convenient material, e.g., platinum or nickel or glass with a platinum coating or gold coating. The spherical connection depicted in this figures represents an embodiment of connectors that are not bonded to at least one of the electrode and IC chip, and in this manner provide a greater degree of freedom of movement for these elements with respect to each other. The spherical contact member is contained with a micromachined feature formed into the electrode **142.** The member can also be contained with a feature formed into the IC chip by methods typically used for the fabrication of MEMS devices. The spherical contact member can also be a conductive, e.g., gold, bump metallurgically bonded to the IC chip. The simplest configuration of this contact method is the formation of a spherical contact surface into the electrode that contacts the IC chip bond pad. For all design variations a reaction force should be applied to maintain contact between the IC chip and the electrode. The assembly can also be over molded with a polymeric material as described in **FIG. 14C. FIG. 14F** show a variation of the contact described in **FIG. 14E****.** The contact is formed on both sides of the IC chip.

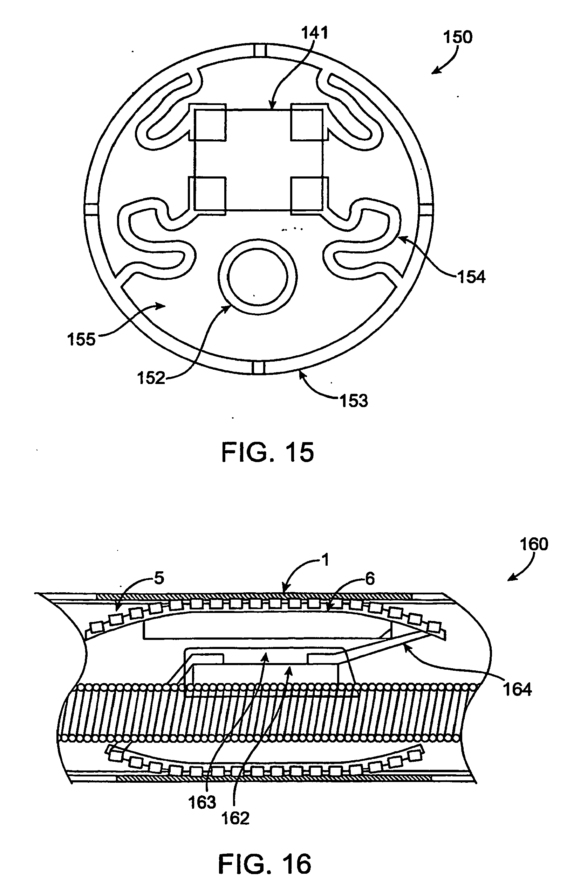

**FIG. 15** shows an alternate orientation of the IC **141** inside the medical device assembly **150.** Connections between the IC and the electrodes **153** is made with the flexible conductive members **154**. The construction of these members has been described previously. A conductive multi-filar coil **152** is shown for scale. The whole assembly is insert molded into the medical device by a flexible polymeric material **155.**

**FIG. 16** shows an embodiment of a flexible electrode assembly **160** that includes a porous flexible polymeric material **161** covering the electrodes, where the porous flexible material contains a pharmacological agent, e.g., a steroid or other agent, in the pores of the material. Following implantation, the steroid leach out of the material over time to reduce pacing thresholds. Shown also is the IC chip **162.** Polymeric encapsulation **163** encapsulates the electrodes to IC chip joint. The flexible conductive member **164** connects the IC chip to the electrode. The flexible electrode **165** is shown with polymeric material **166** backing up the electrode **165.**

An additional connection method between the electrodes employed is certain embodiments is stranded flexible high strength wire or cable. For connections between the satellite and chip to the conductors, one or more conductors can be performed with stranded wires that would be soldered to the chip in the manner described with the electrodes. These would then be formed so they would relieve stresses, and are then wrapped around the conductors and soldered and or metallurgically joined with a process similar to laser welding.

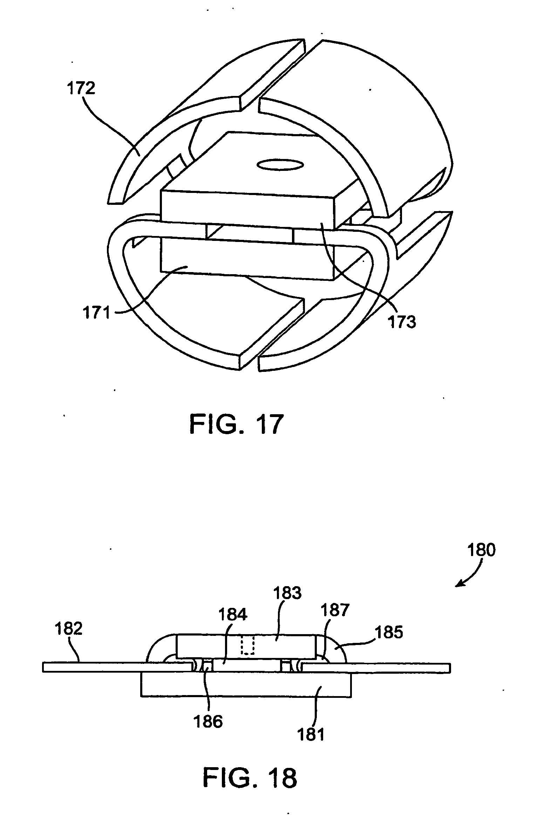

**FIG. 17** shows a final assembly drawing of IC **171,** pressure sensor **173** and electrode **172.** Electrode **172** is connected to IC **171** and pressure sensor **173.** The connection of electrodes and chip could be done by solder bonding, ACF (Anisotropic Conductive Film) or TAB. The typical dimensional scale is from about 1 mm to about 3 mm.

**FIG: 18** shows the cross-section of assembly **180,** including IC **181,** pressure sensor **183,** and electrode **182** connected to IC **181** and pressure sensor **183.** Also provided is cavity **184** of the pressure sensor for pressure sensing. Item **186** serves as spacer (bottom part) and adhesion material (top part). Item **186** controls the final bonding gap between IC **181** and pressure sensor **183.** This gap is bigger than the thickness of electrodes in certain embodiments. Solder **187,** could alternatively be ACF or thermosonic bonding. Under-fill material **185** secures the final assembly.

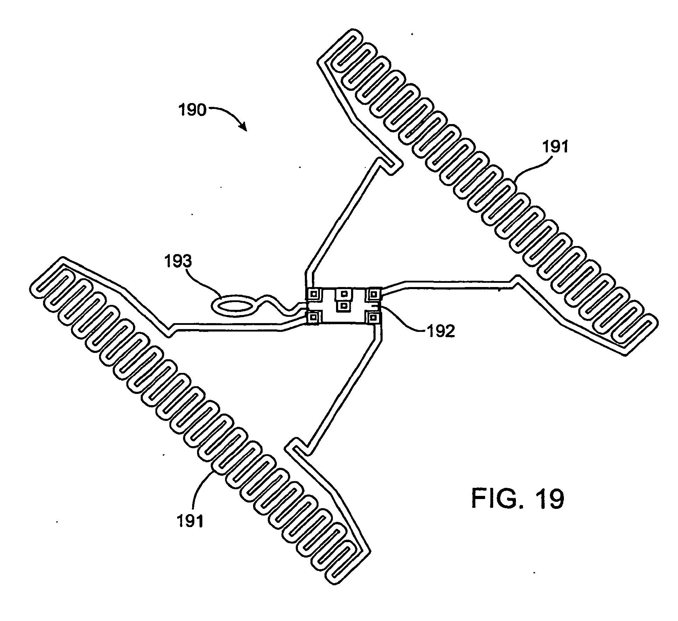

**FIG. 19** shows the electrode pattern at an angle according to an embodiment of the invention. In **FIG. 19****,** structure **190** includes hairpin containing flexible electrodes **191** conductively connected to integrated circuit **192** by flexible connectors **194.** Also shown is flexible connector **193.** As seen in the figure, electrodes **191** are configured at an angle to each other.

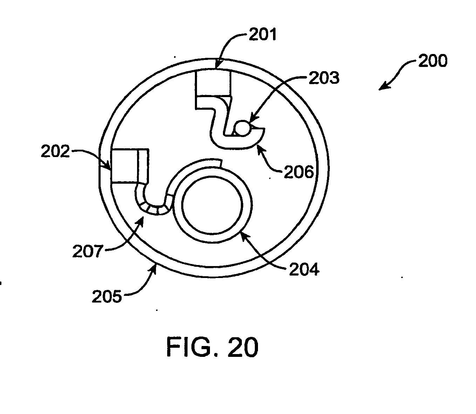

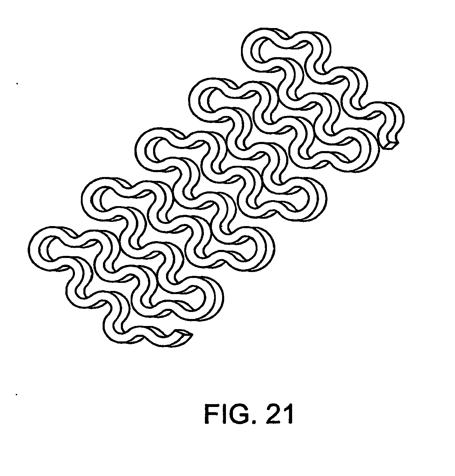

**FIG. 20** shows a cross section of an embodiment of an assembly **200** that includes two ICs **201** and **202,** where one IC **201** handles the high power requirements of the medical device and the second IC **202** handles the lower power requirements for the medical device. The functions are separated due to different processing requirements for the circuits of each IC. Conductor cable **203** connects to one IC with a flexible conductor **206.** Multi-stranded conductor coil **204** connects to the second IC with the flexible conductive member **207.** **FIG. 21** shows detail on the shape of a flexible electrode **210** according to an embodiment of the invention, which shape may be characterized as a stacked serpentine shape. This shape allows the electrode to flex in two axes.

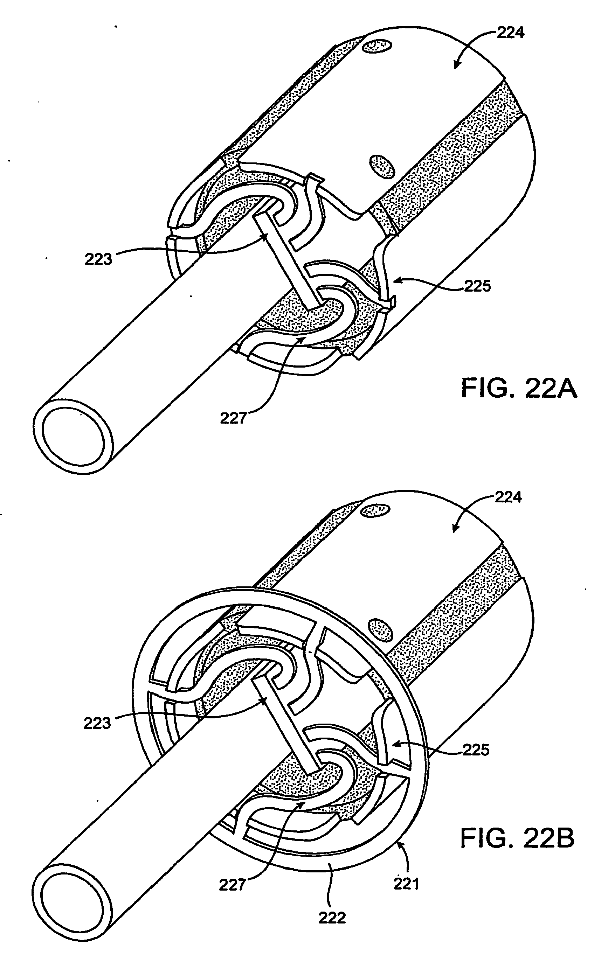

Additional embodiments of the invention that provide certain advantages are depicted in **FIGS. 22** to **25****.** The embodiments disclosed in these figures can be manufactured using protocols that provide for considerably decreased handling, which in turn lowers the physical risk to the IC chip as compared to other manufacturing methods. As a result, the scrap rate is also decreased.

The fatigue resistant IC chip connections of these embodiments enjoy many unique advantages. The entire device is simply and predictably assembled, allowing mass production with limited wastage and lower cost of production. It is unusually suited to robotic automation, rather than the painstaking hand assembly typically required for such devices. Time to assembly is decreased both in robotic and hand assembly embodiments of the present invention. Also, as the final construct is "of a piece," potential material fatigue failures are minimized or eliminated.

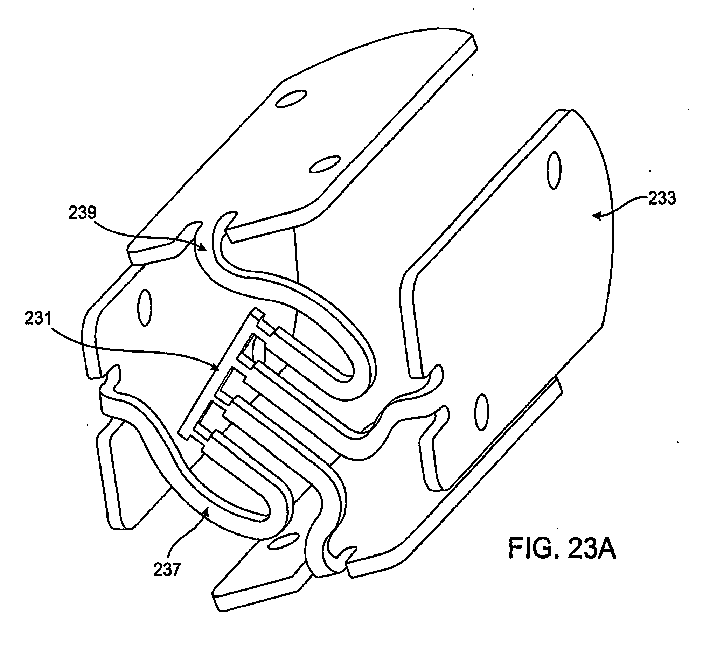

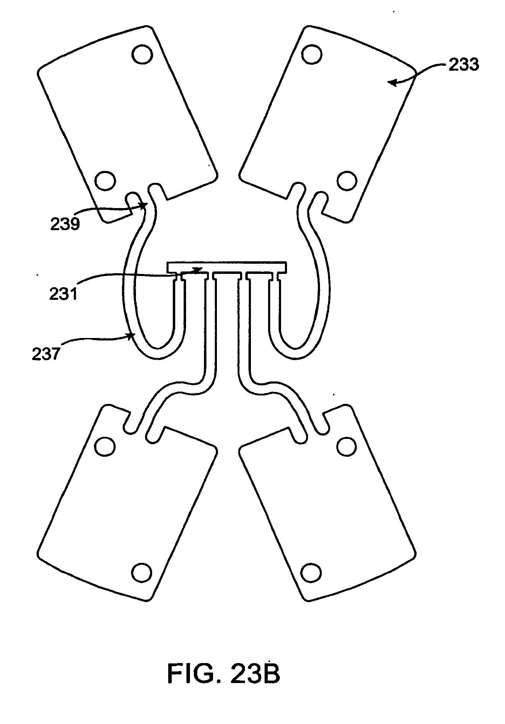

Curved, somewhat flexible, robust attachment of the chip to the electrodes allows for long-term permanent device implantation, features shared with other embodiments of the invention. In one embodiment of the present invention, the attaching "wires" are beveled to provide ease in bending, and a robust final assemblage results, as reviewed in greater detail below. In one embodiment shown in these figures, a highly miniaturized IC chip is soldered together from small pieces. This assemblage is then processed in an oven to flow the solder. After this processing, the device undergoes welding to attach a lead frame to the electrodes and the power is connected to the other side of the chip. This assembly, which is comprised of the lead frame, the IC chip and the power wires, has inserted into its interior the electrodes which have been previously molded to a PEEK ring. The resulting intermediate assembly is then welded to it at the ends. The outer ring falls away, resulting in the finished assembly. See **FIGS. 22A** & **22B****.** In a second embodiment, the steps of attaching the lead frames to the electrodes are eliminated, as will be described below and as shown in **FIGS. 23A** to **23C****.** In this embodiment, the construct is joined, eliminating many of the assemblage and reliability difficulties inherent in the use of welding.

The bending operations in this more advanced embodiment are easier and more reliable than joining operations from the standpoint of electrical conductivity and alignment. The result is better consistency and reliability in the final assemblages. Lower resistances for better current transfer, and basically better communication from the chip to the body are also advantages to this embodiment. An aspect of the present inventive fatigue resistant IC chip connection assembly methods of these embodiments is that the connection very quickly accesses or connects to the IC chip. The methods also provide very quick accesses or connects to the output of the chip to the body, or the chip to a package, or to a circuit or other device before it goes to the body. The invention allows a means to get to the body with a short a path and a minimum of assembly steps from the chip.

**FIGS. 22A** & **B** provide a diagrammatic view of one embodiment of the present invention. A lead frame **221** supports IC chip **223** within quadrant electrode assembly **224.** Lead frame **221** attaches to four electrodes **224.** Element **227** is a bent flexible connection between electrodes **224** and IC **223**. The attachment point **225** between lead frame **221** quadrant electrode assembly **224** is composed of two weldable materials, which are welded together after the frame is fitted into the notch provided in the four electrodes **224.** In one embodiment of the present invention, the method of welding the materials together is laser-welding, which provides a good level of accuracy and predictability. However, stitch or resistance welding as well as soldering or ultrasonic welding are appropriate methods to provide the bond. Selection of a specific bonding method depends on the specifics of the inventive construct with a mind to suitability to that device will optimize the result. Whatever bonding method is selected, when energy is applied, the outer ring substructure **222** falls away from the main assemblage. The purpose of outer ring substructure **222** up to that point is to maintain the alignment of the various structures of lead frame **221** and four electrodes **224.** At the point outer ring substructure **222** falls off, the lead frame **221** and four electrodes **224** become electrically communicative.

**FIGS. 23A** to **23C** provide a simplified embodiment of the present invention as compared to that shown in **FIG. 22****.** In **FIG. 23A****,** the lead frame of **FIG. 22** and four electrodes **224** are incorporated into a single piece via legs **237.** The manufacturing process to produce the construct shown in **FIG. 23A** is simply accomplished by bending the electrodes down with relief **239.** Sacrificial bar **231,** supports the IC chip prior to full assembly. Sacrificial bar **231** keeps the assembly stable during the chip attachment step.

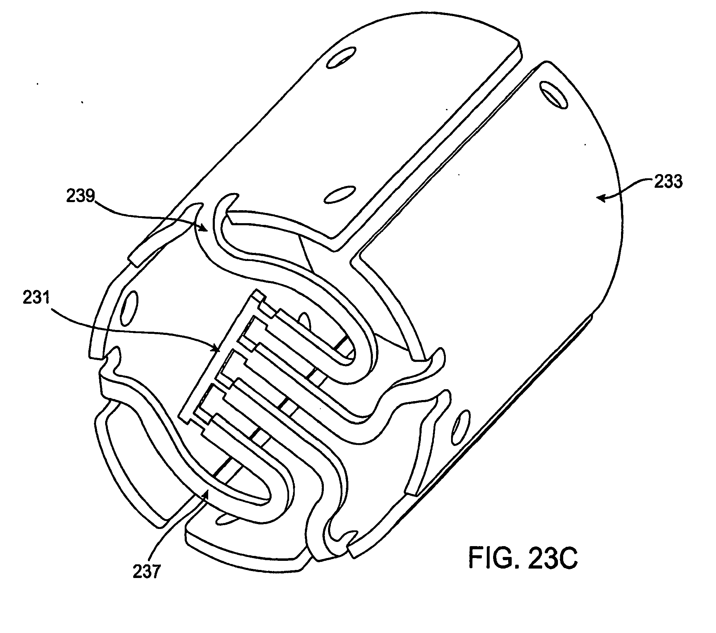

The assembly process for the inventive embodiment in **FIG. 23A** allows the whole device to exist on a single plane until the final stages of manufacture, as shown in **FIG**. **23B**. The final manufacturing stage is when all four electrodes **233** are first bent down at juncture (i.e., relief) **239.** Juncture **239** may be provided with triangular relief cutouts to provide for a smoother, less brittle connection to four electrodes **233.** The final step in molding is shown in **FIG. 23C** where four electrodes **233** are each bent around their long axes to match the curvature of the lead body.

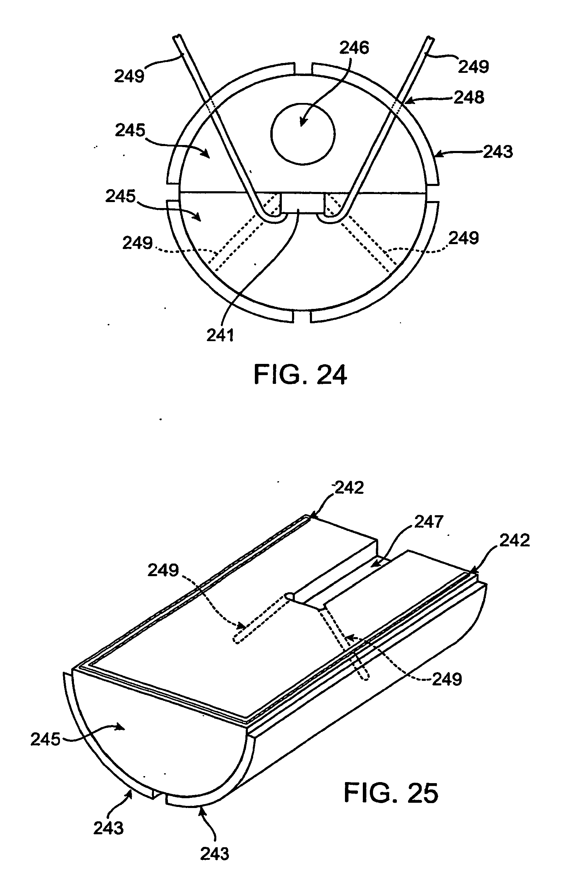

**FIGS. 24** and **25** show a different approach to assembly. In this model, the IC chip is fitted into rectangular notch **247.** Conductive vias **249** run out of rectangular notch **247** to carry the signal from the IC chip to the outside world. This embodiment of the present invention provides a way to seal the IC chip and provide attachments all at the same time. The IC chip within the cylinder contacts pads to make a connection to vias **249.** The construct includes PEEK body **245.** PEEK is a material which has a high-temperature melting point, allowing for soldering and other manufacture protocols. Rectangular notch **247** stabilizes the chip. Four conductive vias **249** are provided, which could be wires. In **FIG. 24****,** four conductive vias **249** are provided. This design embodiment provides a method to seal the IC chip and provide attachments in a single step. Contact pads are provided on the IC chip that are aligned in one of the half-cylinder sections. This assembly provides a simple way to manufacture the inventive device. When PEEK is molten, it has very good adhesive properties which are exploited in one embodiment of the present invention. During manufacture, the PEEK is melted into the platinum electrodes **243.** Two halves of the assembly, each a half cylinder, are manufactured as subassemblies.

The IC chip **241** is placed into rectangular notch **247.** For an ultrasonic welding approach, a raised floss is provided. The sacrificial material **242** provides a good, fluid-tight seal when the two halves are aligned and welded together. This approach is useful to speed the assembly process, because the subassembly will be molded to have the vias and leads **249.**

The IC chip is placed into the in rectangular notch **247** in the cylinder substructure half that will be place over the top of the full assembly. The two aligned halves are held in a clamshell type fixture, clamping the two halves together. Ultrasonic energy is applied, which melts the plastic together.

Sacrificial material **242** is engineered to be sacrificial, that is these pieces are designed to melt. Alternately, sacrificial material **242** can be placed to.fully encircle or be placed inside rectangular notch **247.** As a result, the whole construct is a sealed end, providing maximum hermeticity protection.

Alternately, an opening can be provided. The advantage to having an opening, at some point in the structure, is a place to pass through the power leads to the chip as may be desired. To provide stronger hermeticity protection in this case, it is possible to encapsulate the entire final structure. In the final stages of assembly, the wires have been passed through these vias **248** in **Fig. 24****.** At this stage, the various components can be laser or resistance welded into place. The end of **249** just falls off. Guidewire lumen **246** is shown for orientation to the final device.

The fatigue resistant IC chip connections and assembly methods of the these and other embodiments described herein allow the practicable reproducible production of an IC chip package and attachment design which is uniquely scalable to the necessary dimensions for many medical device applications, such as, but not limited to, intracardiac and intraocular devices, e.g., as reviewed below. The present invention provides for an entire medical device which has the capacity to be scaled to the size of currently available chip-packages alone. This unique miniaturization of a device with robust qualities provides the clinician medical devices of unprecedented applications in their diagnostic and therapeutic armamentarium.

The inventive constructs and assembly methods provide means to get to the body with as short a path as possible from the chip. An important aspect of the present inventive fatigue resistant IC chip connection assembly methods provides very quick accesses or connects to the IC chip. It also provides very quick accesses or connects to the output of the chip to the body, or the chip to a package, or to a circuit or other device before it goes to the body. Though these multiply improved segments of the overall device, the invention allows a means to get to the body with a short as path as possible from the chip.

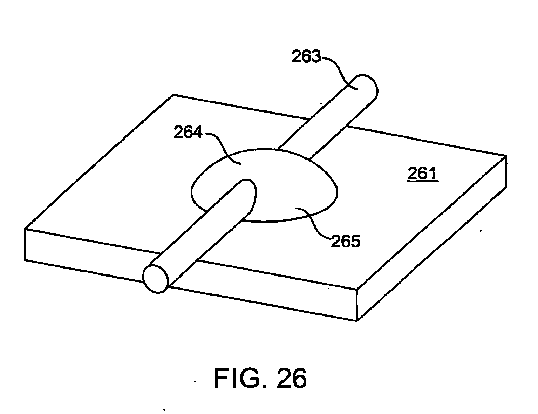

In certain embodiments, the flexible conductive connection is provided by a liquid conductive connector that provides a liquid electrical connection between the IC and electrode components, e.g., as shown in **FIG. 26****.** In **FIG. 24****,** IC **261** is electrically coupled to flexible connector **263** by liquid conductor **262** present in a cap structure **264.** This conductor would serve as a mechanical strain-relief between the two components, ensuring that electrical connection is maintained, regardless of the relative position of the components. Since the conductor is a liquid incapable of supporting a shearing load, the electrical connection is not stressed during any relative displacement of the components during bending of the surrounding packaging or electrical components. This inventive liquid conductor embodiment could come in many different forms. Another inventive embodiment provides a conductive wax with a glass transition temperature just below body temperature could be used to bond the two electrical components together during assembly. After implant, the conductive wax melts and becomes the liquid electrical connection. Similarly, a conductive liquid with a melting point below body temperature could be used. This wax could be a low-melting temperature wax containing a suspension of conductive nanoparticles, e.g., metallic spheres or carbon nanotubes. In an additional embodiment, a low-viscosity conductive hydrogel could be used. This gel would be encapsulated so as to not dry out during storage or use. In certain embodiments, a bracket may be used to further secure the liquid conductor to the surface of the IC.

In certain embodiments, a compliant and electrically conductive adhesive is employed that includes a high-aspect ratio conductive member, e.g., carbon nanotube, present in a suitable flexible carrier material, e.g., silicone rubber. Both components are biocompatible and the carbon nanotubes can be functionalized to promote or hinder the absorption of proteins onto the carbon structure to alter the human body's reaction to the carbon nanotube. The importance of using carbon nanotubes is that the high-aspect ratio structure ensures electrical conductivity during elastic deformation of the material. The carbon nanotubes "threads" can distort but still provide electrical connection. Additionally, the silicone can be mixed with a much lower weight percentage of carbon nanotubes.

As summarized above, certain embodiments of the subject structures are characterized by having shaped IC chips which impart fatigue resistance properties to the structure. Embodiments of such structures are now reviewed in more detail in terms of the figures.

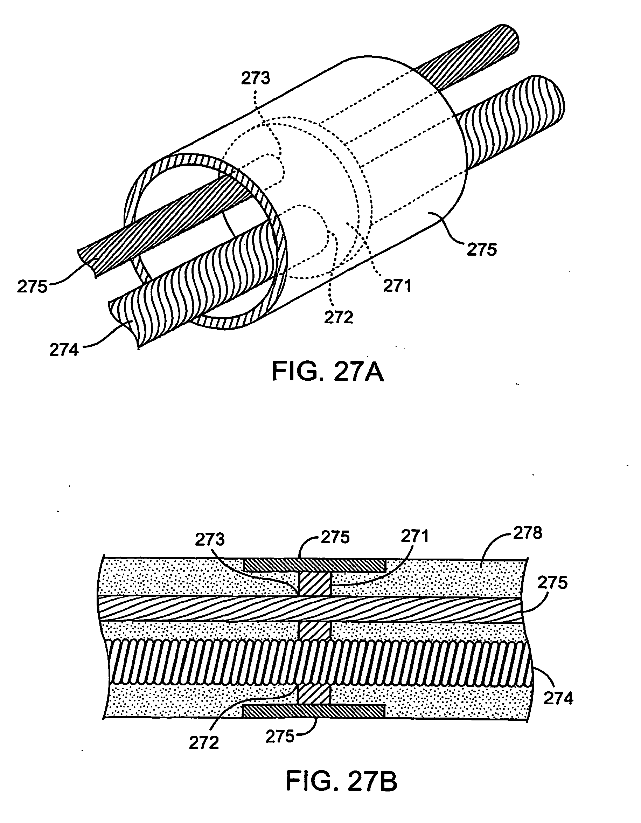

**FIG. 27A** shows one embodiment of the present inventive IC chip forms. In this embodiment, an integrated circuit (IC) chip **271** is formed into a non-rectangular shape, e.g., a round shape. Other shapes can include ovoid, elliptical, partially rounded, eccentrically configured, squared of on one or more corners, and the like. These and many other variety of forms provide unprecedented advantages for incorporation into a medical device. The IC chip **271** pictured shown in **FIG. 27** is provided with holes **272** and **273** through its structure. These orifices allow for connection to conductors **274** and **275** as well as the passage of other medical device and tools, and can be custom designed to fit appropriate forms. Fluids would also be able to pass through the IC chip when it is incorporated into a medical device. The IC chip **271** is attached to ring electrode **276.** **FIG. 27B** shows a cross-section view of the IC chip **1** connected to an electrode **276,** where the structure is present in medical lead **278.** Two holes, **272** and **273,** are shown in the IC chip **271.** However, the design can incorporate several holes in the chip in various arrangements, as desired. The holes are also not required to be round, but may be selected from a varieties of shapes most appropriate to the need, which will be readily understood by the ordinary skilled artisan.

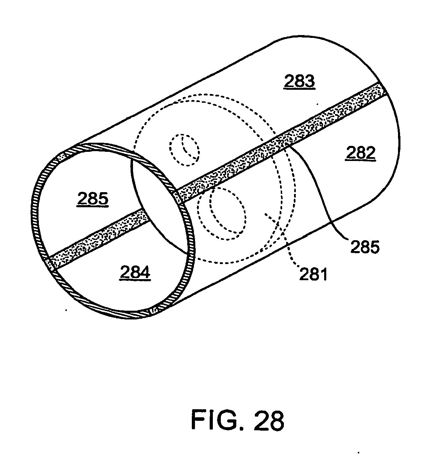

**Fig. 28** shows IC chip connected to a multiplicity of electrodes, i.e., **281, 282, 283** and **284,** where the electrodes are arranged in a quadrant configuration. The electrodes are connected to IC chip by solder **285** in this representation. However, other electrical connection methods are useful within the scope of this design. The electrodes are sized and positioned based on clinical requirements. This configuration allows a unique mass production method for the chip. The electrodes are embedded in an extended cylindrical shape. The surface is then polished, and the face cut.

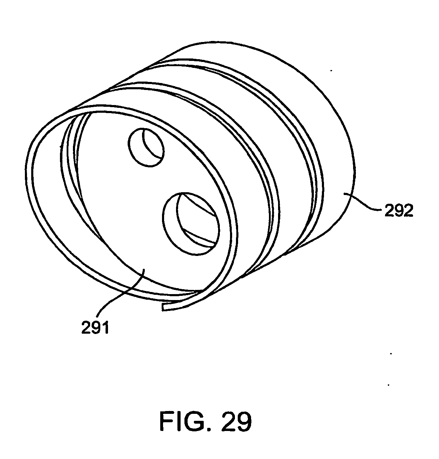

**FIG. 29** shows a coil configuration for electrode **292** connected to IC chip **291.** This configuration provides a method to reduce the stress concentrations at the location of the IC chip in the device due to the flexibility of the coil. The potential for material fatigue based failure is substantially reduced by this configuration.

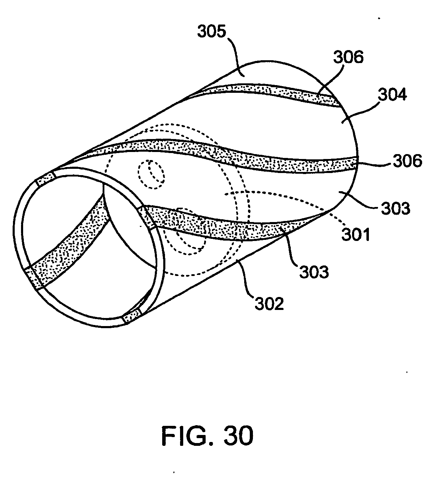

**FIG. 30** (informal figure 4) describes IC chip **301** that is attached to electrodes **302, 303, 304** and **305.** The electrodes are supported by polymer **306.** The polymer **306** can be PEEK, PEKK, polyamide, ETFE, urethane, or other suitable material. The material may also be a ceramic material, alumina, silicon carbide or other suitable material. Embedding the electrodes in this manner provides many advantages, such as securing them in place, protecting them against possible biological fluid challenges, and providing a flexible support to cushion against impact forces. The electrodes reconfigured in a helix in this representation, but can take other forms as well.

**FIG. 31** describes IC chip **311** connected to electrodes **312, 313, 314** and **315** that are dispersed along the length of the medical device. In this inventive configuration, two of the electrodes **312, 315** are more distal from IC chip **311,** while two of the electrodes **313,314** are more proximal from IC chip **311.** This form of configuration provides the opportunity for larger features to be accommodated within the medical device. It also disperses the strain, and provides for more flexibility than might otherwise be available. Additional, flexibility can be customized along the length of the device to provide optimum variable rigidity, such as may be required when accessing the coronary sinus.

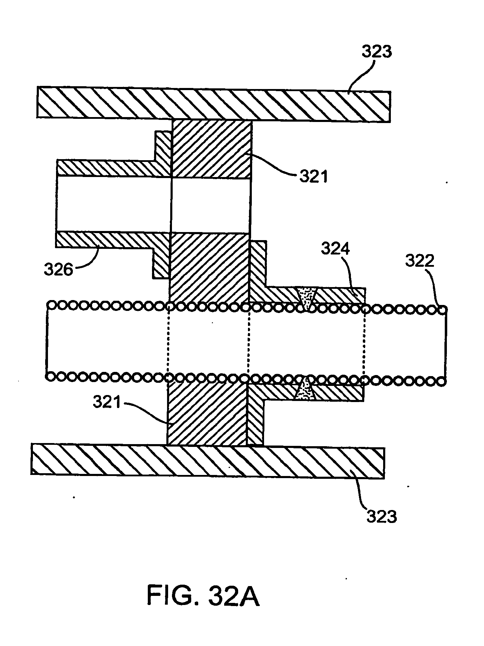

**FIG. 32A** describes IC chip **321** connected to electrodes **323.** The electrical connection from IC chip **321** to the device is through metallic coil **322** that is welded or bonded to a metallic flange **324.** Metallic coil **322** can take a number of configurations, such as a single conductor wound as a coil. The coil can be insulated with ETFE, polyimide, or other suitable material. The insulation is then stripped where the electrical connection is made with flange **324.** The coil can also be a multi-filar conductor where electrical connection can be made with only a fraction of the insulated filar conductors. Two of six strands can be connected for example. The conductor coil can provide a central lumen for the passage of a guidewire or fluids through the medical device. The coil can have a liner of PTFE or urethane to provide isolation from the coil to the device or fluid in the lumen. **FIG. 32A** also shows flange **326** on the opposite side of chip **321** to allow for connection with additional conductors. The conductors can be coils as described previously, cables, or other suitable forms. The conductor materials can be MP35N, stainless steel, platinum, titanium, tantalum or other appropriate materials. The conductors can have conductive center materials made from silver, copper or gold.

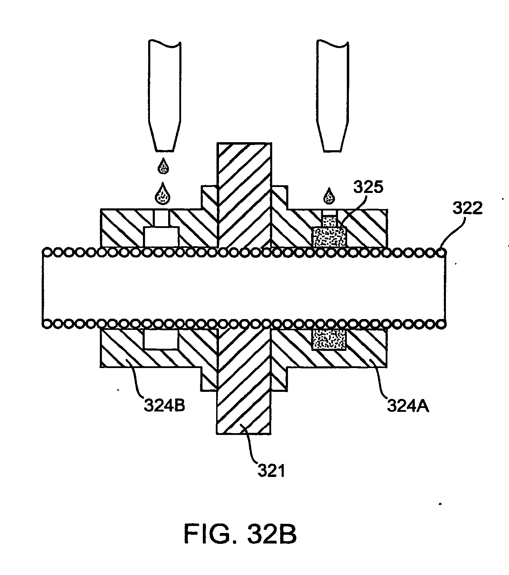

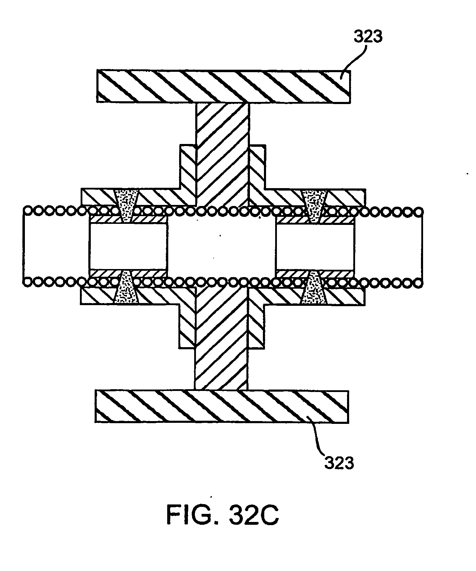

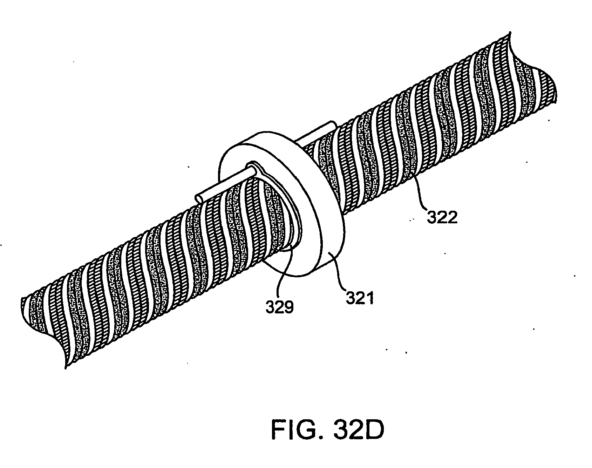

**FIG. 32B** describes two flanges **324A, 324B** on each side of the IC chip **321** electrically connected to a conductive coil **322** with a flexible conductive polymer **325.** This configuration allows stability with substantial flexibility. The conductive polymer can be made conductive with the addition of carbon in the form of flakes or nanotubes. Silver or platinum flakes can also be added to increase the conductivity. The polymer can be a silicone, urethane or epoxy, or other suitable materials. If desired, the electrical connection can be increased with the addition of a laser or spot weld in addition to the conductive material. The connection with the flanges can be accomplished with a suitable solder, such as Pt-Sn, Pt-Ge, Au-20Sn, Au-19.5Si, Au-Ge, or Sn-5Ag or other materials which are relatively biocompatible and corrosion resistant. **FIG. 32C** provides another depiction of the structure shown in **FIG. 32B****,** and also shows the electrodes **323.** **FIG. 32D** describes a metallic band **329** under the flange and coil **322.** The flange is attached to IC chip **321** as described in previous figures. The coil flange and band are welded together.

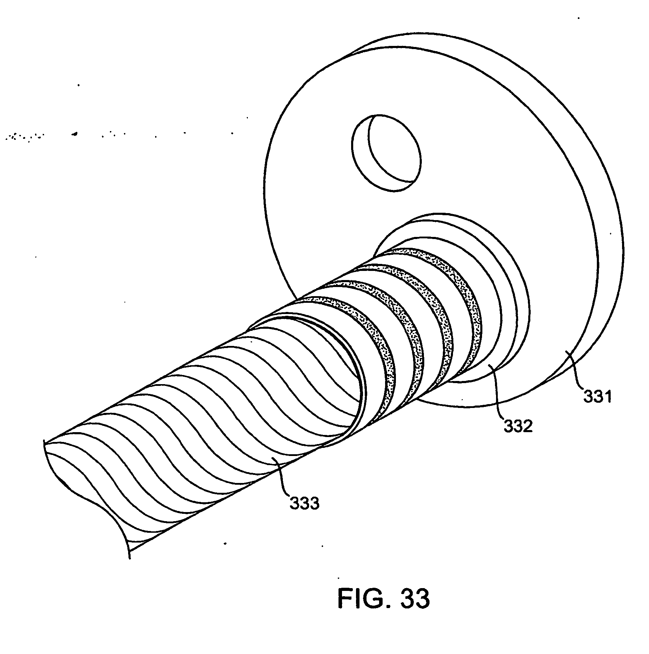

**FIG. 33** describes a flange **332** that is attached to an IC chip **331** as described earlier. The flange is laser cut, EDM'ed or electrochemically machined to form a flexible structure that reduces the bending stresses applied to the IC chip. Also shown is coil conductor **333.**

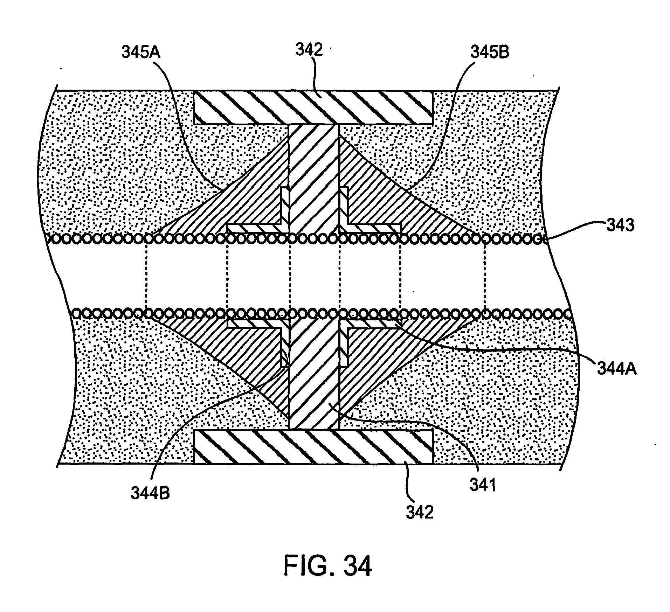

**FIG. 34** describes an IC chip **341** that is attached to electrodes **342** with electrical cable **343** running through the IC chip. An elastomer boot **344A, 344B** is molded at either end of the chip **341** to reduce the bending stresses applied to the chip. The boot or strain relief is then over-molded with higher elongation elastomer **345A, 345B** to form the balance of the body of the device.

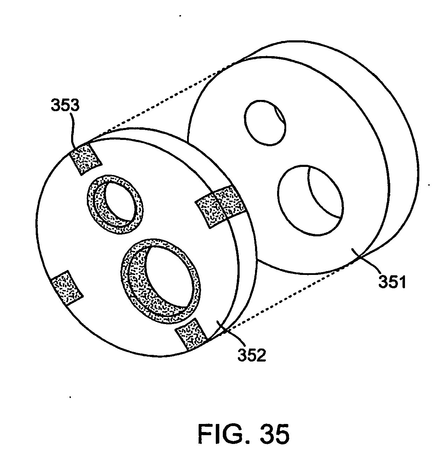

**FIG. 35** describes a formed structure **352** of a polymer or ceramic with Pt or other suitable material formed into the structure. The assembly is then sawn or laser cut to 0.05 to .1 mm thick. The metallic portion **353** of the assembly **352** matches the location of bond pads on the IC chip **351** described previously. The assembly is bonded to the IC chip with a conductive material at the bond pad location. The electrode described previously is welded or bonded to this assembly which shields the IC chip from stresses applied to the medical device.

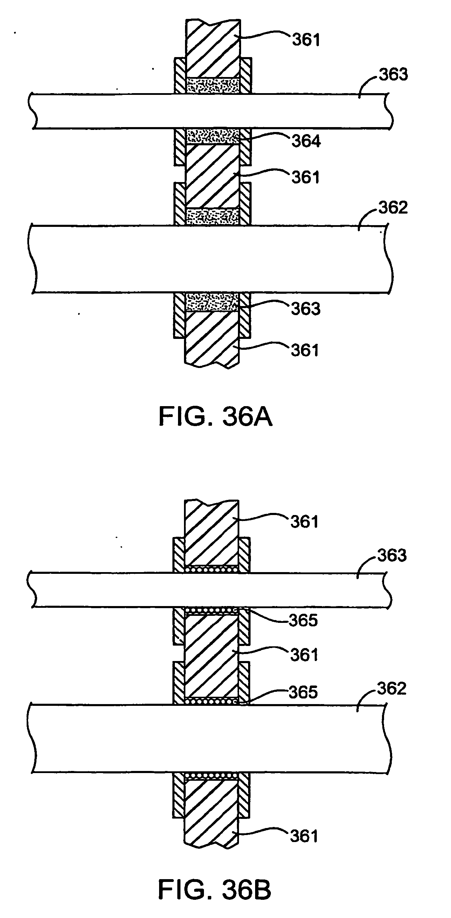

**FIGS. 36A** & **36B** describe a flexible connection from the conductors **362, 363** to the IC chip **361.** The electrical connections **363, 364** are made with conductive polymer such as a carbon nanotube filled silicone, epoxy or thermoplastic such as PEEK. The conductive material could also be a conductive gel or an assembly of conductive structures, e.g., balls, e.g., as shown as element **365** in **FIG. 36B**. Alternatively, nanofiber suspended in a conductive fluid or a ferrofluid with a magnetic material can be utilized, e.g., as described above.

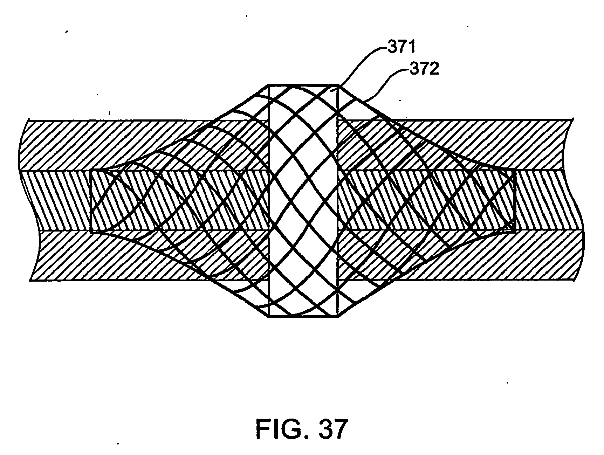

**FIG. 37A** provides a view of an embodiment of an electrode that is attached to the IC chip **371.** The electrode **372** is a Pt or other mesh. The electrode mesh may be aligned to the IC chip or alternately attached to the IC chip at some angle.

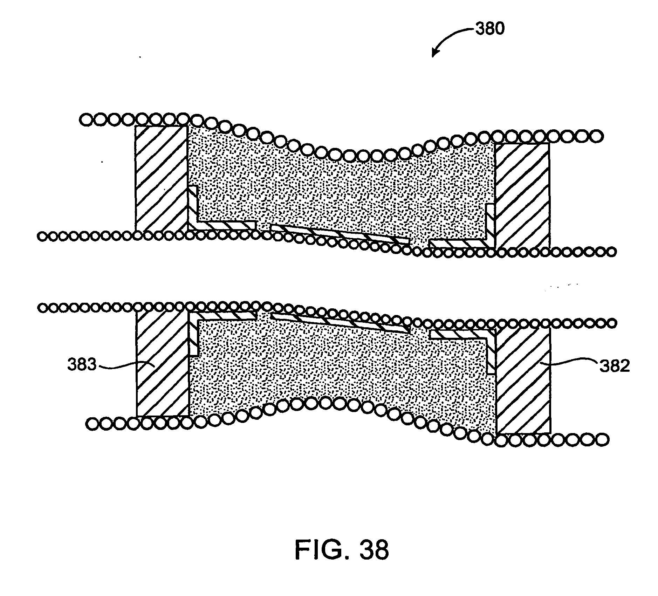

**FIG. 38** shows an embodiment of a device according to the invention in which a fiber reinforced medical device **380** has a fiber **381** braded along the section of the device with the IC chips **382, 383.** In this case, the device would be of variable diameter. Again, this diameter can provide important dimensions optimal for device placement and anchoring. In an alternative configuration, the fibers are wound directly onto the medical device. Alternately the fibers can be provided from strips cut from fabric and wound on to the medical device. In this inventive embodiment, the fiber follows the contour of the device. The device can have a uniform diameter or the device can have sections of larger diameters at the locations of the electrodes. The larger diameter of the device at the electrode locations helps to insure electrical contact the tissue. The larger diameter sections of this inventive embodiment also help to anchor the device in small compliant vessels. The device is inserted into a vein or artery with the vessel stretching at the electrode locations. The tip of the device can also have a larger diameter section that tapers from small to larger diameter like a cone. The shape would then quickly transition back to a smaller diameter moving more proximally down the lead. The cone could also have soft thread forms applied to the cone. The cone at the tip of the device could also be a location for an IC chip and electrode set.

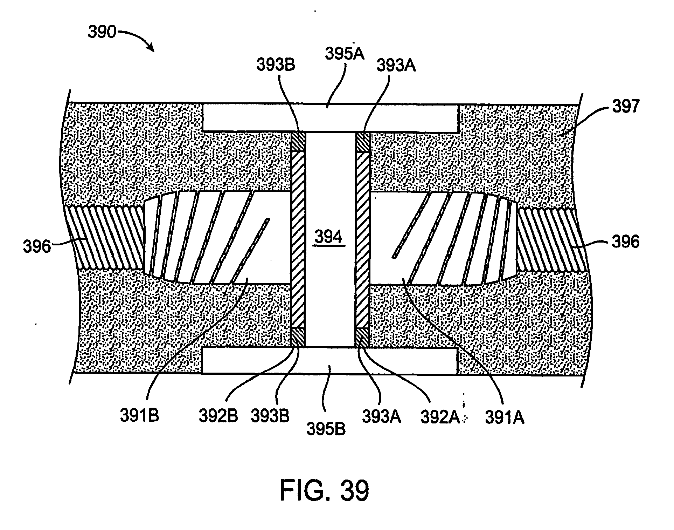

**FIG. 39** provides a view of a multi electrode/IC device according to another embodiment of the invention. Structure **390** is comprised of a pair of spiral cut sleeves **391A** and **391B** with flanges **392A** and **392B** which provides a means to mechanically support the chip **394**; electrically connect the **chip 394** to the conductor coil **396;** and provide strain relief between the chip **394** and the conductor coil **396.** While the figure shows an embodiment with only one conductor coil, other embodiments of the inventive device include multiple conductor coils, e.g., where each coil has its own pair of spiral cut sleeves. The spiral cut sleeve **391A** and flange **392A** are one piece which is made of a medical implantable grade metals such as platinum-iridium. The outer rim **393A, 393B** of the flange is made of a non-conductive medical implantable grade material such as PEEK and insulates the conductive material of the flange **392A** and the electrodes **395A, 395B** on the surface of the lead body **397.** In order to provide mechanical support to the chip **394,** the stiff flanges **392A, 392B** are bonded on both sides of the chip **394** and then bonded to the electrodes **395A, 395B.** The flanges **392A, 392B** act as the primary structural elements holding the electrodes **395A, 395B** in place relative to the conductor coil **396** and lead body **397** while the sandwiched chip **394** is essentially isolated from mechanical loading. In order to create a reliable electrical contact between the chip **394** and the conductor coil **396,** the metal flanges **392A, 392B** are electrically connected to the chip **394** using any convenient technique, such as soldering or laser welding. In turn the metal spiral cut sleeves **391 A, 391B** are soldered or laser welded to the conductor coil **396.** Multiple redundant laser welds or solder points can be used to increase the reliability of the electrical contact between the chip **394** and flanges **392A, 392B,** and spiral cut sleeves **391A, 391B** and conductor coil **396.** In order.to provide strain relief between the chip **394** and the conductor coil **396** a spiral cut sleeve **391A, 391B** is used. The spiral cut sleeve **391A**, **391B** can be manufactured using standard laser cutting techniques. The spiral cut adds flexibility to the sleeve allowing it to bend with the conductor coil **396** thereby reducing the stress concentrations which would arise at the interface between a bending conductor coil **396** and stiff solid sleeve. Optimized strain relief using the spiral cut sleeve **391A, 391B** is achieved by varying the pitch and width of the spiral cut and if desired by tapering the distal ends of the sleeve with the goal of creating a seamless stiffness transition via the sleeve between the very flexible coil **396** and the stiff flanges **392A, 392B.**

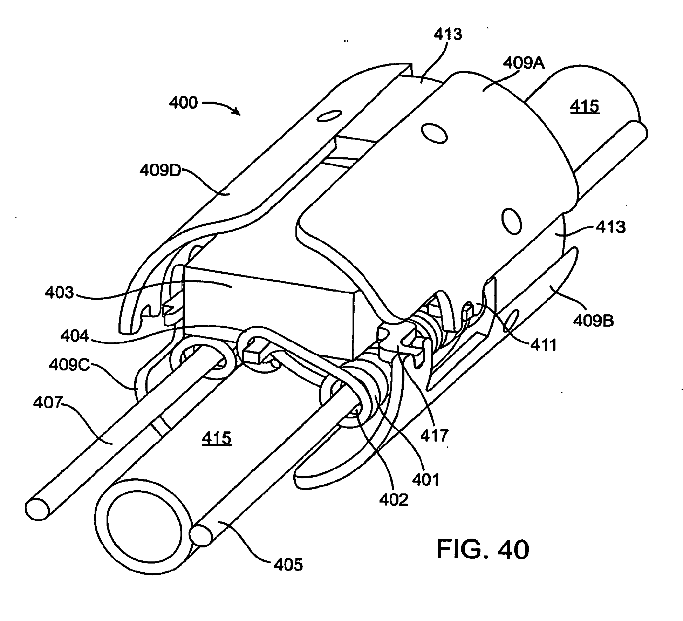

**FIGS. 40** to **44** provide a depiction of yet another embodiment of the subject segmented electrode structures in which electrical connections are provided by coils. In the embodiment depicted in these figures, a coiled spring is provided to attach and provide electrical communication between the IC and one or more elongated conductive members. Compression and stretching forces in the directions of the length of elongated conductive members as in relation to the chip-electrode assembly can lead to strain on attachment to the chip. The use of a spring provides a source of relief for this tension, limiting the strain on the connection. In some cases, the spring may be tapered, providing a graduated transition of the strain. This will limit the impact of a strain in that dimension on the attachments.

In one embodiment of the present invention, a flexible spring is used to provide stress reduction on electrical connections. The spring can be made from many appropriate materials, including but not limited to: platinum, platinum iridium, platinum nickel, platinum tungsten, MP35N, Elgiloy, L605, 316 stainless steel, titanium, nickel titanium, Nitinol, cobalt chromium, cobalt, NiTi, tantalum, among other appropriate material choices.

The flexible spring of the present invention is provided at a length most appropriate to the particular miniaturized device and its application. This can potentially be as long as the device of which it is a part. By example, the length of the spring can be about 0.080 to about 0.200 inches, such as from about 0.030 to about 0.100 inches, and including from about 0.015 to about 0.250 inches. The wire diameter of the spring will be selected as appropriate to the material and as to the particular application. Wire diameter ranges for some embodiments of the present invention are about 0.0005 to about 0.020 inches, such as from about 0.002 to about 0.010 inches, and including about 0.003 inches.

Pressures on the device may also occur as the elongated conductive members curve away from or curve towards the electrode, e.g., quadrant electrode, assembly in either a sideways or up and down directions. These compression and extension forces again can be relieved by the use of the inventive flexible attachment structure, and other stress relief features working synergistically to more rigid structures of the device.

**FIG. 40** shows an assembly **400** with flexible connections, in this case, a micro-spring used as part of the assembly. Various other flexible connectors can be employed, as desired. As shown in **FIG. 40** flexible connections **401** are provided between IC **403** and elongated conductive members **405** and **407.** This design creates a flexible connection between the IC and the elongated conductive members. In this design embodiment, the elongated conductive members **405** and **407** are placed into inner lumen **402** of flexible connections **401,** as shown in the assembly.

IC **403** is attached to quadrant electrodes **409A, 409B, 409C** and **409D** by junctures **411.** Quadrant electrodes **409A, 409B, 409C** and **409D** are joined together with PEEK material **413.** Guide wire lumen **415** runs beneath IC **403** and beneath and/or between elongated conductive members **405** and **407,** all running through or contained with quadrant electrodes **409A, 409B, 409C** and **409D.**

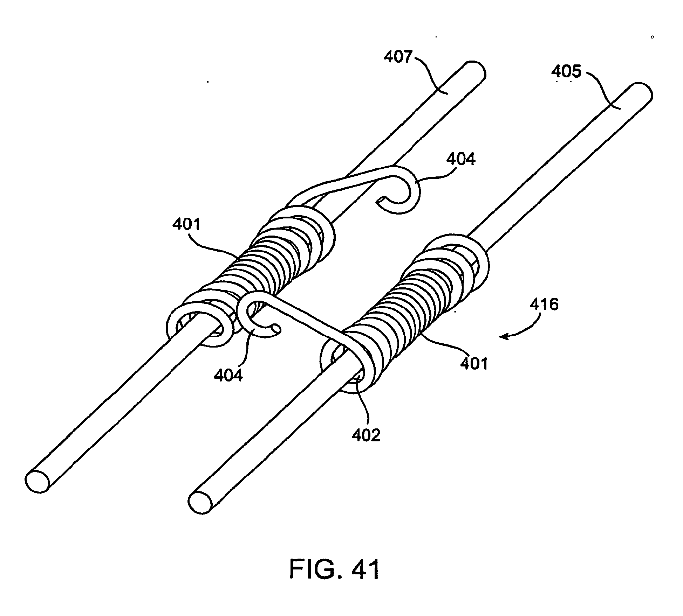

**FIG. 41** provides a view of a first sub-assembly of the final assemblage shown in **FIG. 40****.** In this sub-assembly, elongated conductive members **405** and **407** are placed into inner lumen **402** of flexible connections **401.** Spring fingers **404** are provided for later physical and electrical attachment of flexible connections **401** to IC **403.** This point in the assembly is a convenient time for the flexible connections **401** to be provided with an attachment to the elongated conductive members **405** and **407.** This can be accomplished by a number of methods, e.g., the spring can be crimped to the elongated conductive members **405** and **407**, shown here as crimped area **416.** Flexible connections **401** can also be designed to have an interference fit with the elongated conductive members **405** and **407** (not shown). Any convenient attachment approach can be employed that intimate electrical contact with the cable is preferable.

In certain embodiments of the present invention, the area from the beginning of the coil of flexible connections **401,** the closely wrapped section is tapered. This design feature facilitates the assembly process. It also provides a portion of the spring that can expand and contract in the axial direction. The physical challenge of axial expansion and contraction can occur if there is motion between the elongated conductive members **405** and **407** and IC **403.** Additional, axial expansion and contraction can occur with more universal stresses upon the device as a whole.

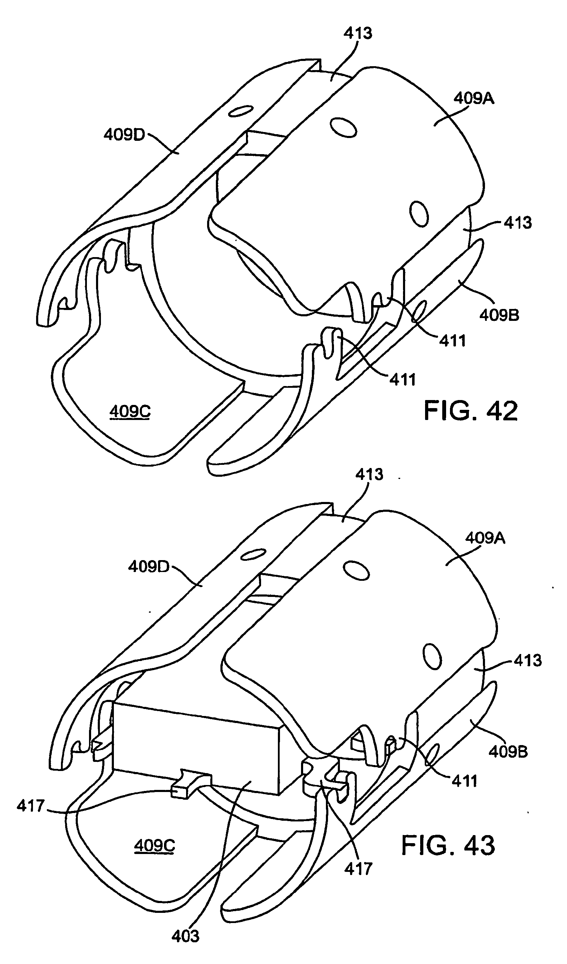

**FIG. 42** illustrates a second subassembly with quadrant electrodes **409A, 409B, 409C** and **409D** molded together with PEEK material **413.** This second subassembly provides an intermediated structure where quadrant electrodes **409A, 409B, 409C** and **409D** are fully joined in a single, but fatigue resistant structure. The junctures **411** facilitate and stabilize later attachment of quadrant electrodes **409A, 409B, 409C** and **409D** to IC **403,** as is also shown.

**FIG. 43** illustrates a third subassembly, introducing IC **413** to the assembly. In this case, IC **413** is provided with attachment tabs **417** to expedite the joining of IC **413** to quadrant electrodes **409A, 409B, 409C** and **409D.** IC **413** is introduced into the lumen of quadrant electrodes **409A, 409B, 409C** and **409D.** The attachment tabs **417** of quadrant electrodes **419** are positioned into the junctures **411** of IC **413.** Typically, the attachment tabs **417** and junctures **411** are welded to provide further stability. In this way, a direct connection of IC **413** quadrant electrodes **409A, 409B, 409C** and **409D** is achieved.

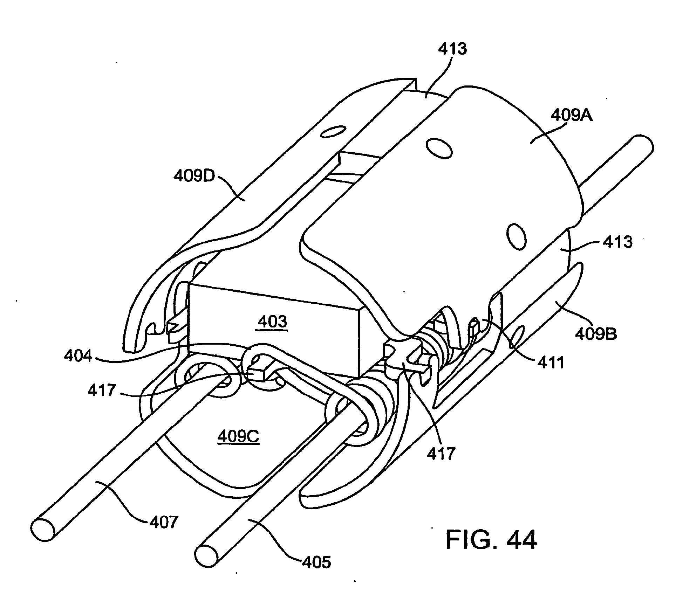

**FIG. 44** illustrates a fourth subassembly. In this view, the subassembly shown in **FIG. 41** is introduced into the subassembly shown in **FIG 43****.** Elongated conductive members **405** and **407** are in inner lumen **402** of the flexible connections **401.** This subassembly is situated within the half cylinder between IC **403** attached to quadrant electrodes **409A, 409B, 409C** and **409D.** The finger **404** of flexible connections **401** allows attachment of flexible connections **401** directly to IC **403.** As with the attachment of some of the above mentioned components, typically the attachment flexible connections **401** to the IC **403** is welded to increase stability.

The final resulting assembled device shown in **FIG. 40** enjoys many advantages provided by its various parts and features. By example, the flexible connections **401** provide a fault resistant connection, even in a highly challenging environment such as the heart. The PEEK material **413** joining quadrant electrodes **409** provides structural stability, especially during the subassembly joining. These design innovations assure fatigue resistance and stress reduction of the device without compromising its structural integrity.

Working synergistically with the more fatigue resistant members of the construct, joined areas, such as junctures **411** which can include welding, providing a basic, strong architectural integrity to the device. Such features as attachment tabs **417** assure that these joined portions of the device are well aligned, and also provide additional structural stability, decreasing strain on the weld junctures.

### DEVICES AND SYSTEMS

Aspects of the invention include devices and systems, including implantable medical devices and systems that include the hermetically sealed structures according to embodiments of the invention. The devices and systems may perform a number of different functions, including but not limited to electrical stimulation applications, e.g., for medical purposes, analyte, e.g., glucose detection, etc.

The implantable medical devices and system may have a number of different components or elements in addition to the electrodes, where such elements may include, but are not limited to: sensors (e.g., cardiac wall movement sensors, such as wall movement timing sensors); processing elements, e.g., for controlling timing of cardiac stimulation, e.g., in response to a signal from one or more sensors; telemetric transmitters, e.g., for telemetrically exchanging information between the implantable medical device and a location outside the body; drug delivery elements, etc. As such, the subject hermetically sealed structures may be operably coupled, e.g., in electrical communication with, components of a number of different types of implantable medical devices and system, where such devices and systems include, but are not limited to: physiological parameter sensing devices; electrical (e.g., cardiac) stimulation devices, etc.

In certain embodiments of the subject systems and devices, one or more segmented electrode structures of the invention are electrically coupled to at least one elongated conductive member, e.g., an elongated conductive member present in a lead, such as a cardiovascular lead. In certain embodiments, the elongated conductive member is part of a multiplex lead, e.g., as described in Published PCT Application No. WO 2004/052182 and US Patent Application No. 10/734,490, the disclosure of which is herein incorporated by reference. In some embodiments of the invention, the devices and systems may include onboard logic circuitry or a processor, e.g., present in a central control unit, such as a pacemaker can. In these embodiments, the central control unit may be electrically coupled to one or more hermetically sealed structures via one or more conductive members.

Devices and systems in which the subject segmented electrode structures find use include, but are not limited to, those described in: WO 2004/066817 titled "Methods And Systems For Measuring Cardiac Parameters"; WO 2004/066814 titled "Method And System For Remote Hemodynamic Monitoring"; WO 2005/058133 titled "Implantable Pressure Sensors"; WO 2004/052182 titled "Monitoring And Treating Hemodynamic Parameters"; WO 2004/067081 titled "Methods And Apparatus For Enhancing Cardiac Pacing"; US8123684 entitled "Methods and Systems for Programming and Controlling a Cardiac Pacing Device" filed 12/23/04; U.S. Patent titled "Fiberoptic Cardiac Wall Motion Timer" filed 3/3/05; U.S. Patent titled "Cardiac Motion Detection Using Fiberoptic Strain Gauges," filed 3/31/05; U.S. Patent titled "de Minimus Control Circuit for Cardiac pacing and Signal Collection," filed 5/9/05; U.S. Patent titled "Deployable Epicardial Electrode and Sensor Array," filed 8/8/05; U.S. Patent titled "Electrical Tomography" filed 8/5/05; U.S. provisional patent application no. 60/707995 titled "Methods and Apparatus for Tissue Activation and Monitoring" filed 8/12/05; U.S. Patent US 7983751 titled "Measuring Conduction Velocity Using One or More Satellite Devices," filed /12/05.

Some of the present inventors have developed Doppler, pressure sensors, additional wall motion, and other cardiac parameter sensing devices, which devices or at least components thereof can be present in medical devices according to embodiments of the invention, as desired. Some of these are embodied in currently filed provisional applications; "One Wire Medical Monitoring and Treating Devices", U.S. Patent filed 09/02/2004, U.S. Patent titled "Pressure Sensors having Stable Gauge Transducers"; U.S. Patent "Pressure Sensor Circuits"; U.S. Patent titled "Pressure Sensors Having Transducers Positioned to Provide for Low Drift"; U.S. Patent titled "Pressure Sensors Having Neutral Plane Positioned Transducers"; U.S. Patent titled "Implantable Pressure Sensors"; U.S. Patent titled "Pressure Sensors Having Spacer Mounted Transducers"; "Stable Micromachined Sensors" U.S. Patent filed 09/30/04, "Amplified Complaint Force Pressure Sensors" U.S. Patent filed 10/06/04, "Cardiac Motion Characterization by Strain Measurement" U.S. Provisional Patent Application filed 12/20/04, and PCT Patent Application entitled "Implantable Pressure Sensors" filed 12/10/04, "Shaped Computer Chips with Electrodes for Medical Devices" U.S. Patent filed 2/22/05; "Fiberoptic Cardiac Wall Motion Timer" U.S. Patent filed 3/03/2005; "Cardiac Motion Detection Using Fiberoptic Strain Gauges" U.S. Provisional Patent Application 60/ 667,749 filed 3/31/05.

In certain embodiments, the implantable medical devices and systems which include the subject segmented electrode structures are ones that are employed for cardiovascular applications, e.g., pacing applications, cardiac resynchronization therapy applications, etc.

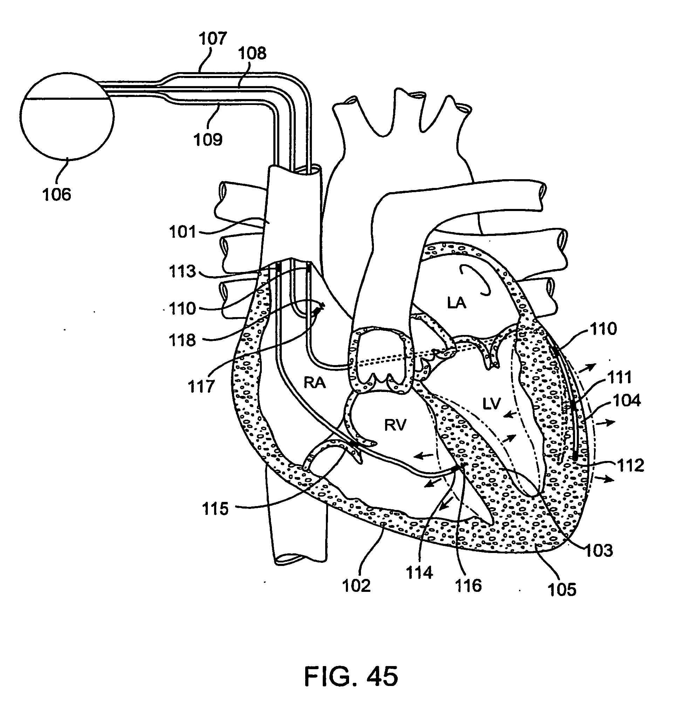

A representative system in which the hermetically sealed integrated structures find use is depicted in **FIG. 45****,** which provides a cross-sectional view of the heart with of an embodiment of a cardiac resynchronization therapy (CRT) system that includes hermetically sealed integrated circuits according to embodiments of the invention. The system includes a pacemaker can **106,** a right ventricle electrode lead **109,** a right atrium electrode lead **108,** and a left ventricle cardiac vein lead **107.** Also shown are the right ventricle lateral wall **102,** interventricular septal wall **103,** apex of the heart **105,** and a cardiac vein on the left ventricle lateral wall **104.**

The left ventricle electrode lead **107** is comprised of a lead body and one or more electrode assemblies **110,111,** and **112.** Each of the electrodes includes a hermetically sealed integrated circuit. Having multiple distal electrode assemblies allows a choice of optimal electrode location for CRT. In a representative embodiment, electrode lead **107** is constructed with the standard materials for a cardiac lead such as silicone or polyurethane for the lead body, and MP35N for the coiled or stranded conductors connected to Pt-Ir (90% platinum, 10% iridium) electrode assemblies **110,111** and **112.** Alternatively, these device components can be connected by a multiplex system (e.g., as described in published United States Patent Application publication nos.: 20040254483 titled "Methods and systems for measuring cardiac parameters"; 20040220637 titled "Method and apparatus for enhancing cardiac pacing"; 20040215049 titled "Method and system for remote hemodynamic monitoring"; and 20040193021 titled "Method and system for monitoring and treating hemodynamic parameters; to the proximal end of electrode lead **107.** The proximal end of electrode lead **107** connects to a pacemaker **106.**

The electrode lead **107** is placed in the heart using standard cardiac lead placement devices which include introducers, guide catheters, guidewires, and/or stylets. Briefly, an introducer is placed into the clavicle vein. A guide catheter is placed through the introducer and used to locate the coronary sinus in the right atrium. A guidewire is then used to locate a left ventricle cardiac vein. The electrode lead **107** is slid over the guidewire into the left ventricle cardiac vein **104** and tested until an optimal location for CRT is found. Once implanted a multi-electrode lead **107** still allows for continuous readjustments of the optimal electrode location.

The electrode lead **109** is placed in the right ventricle of the heart with an active fixation helix at the end **116** which is embedded into the cardiac septum. In this view, the electrode lead **109** is provided with one or multiple electrodes **113,114,115.**

Electrode lead **109** is placed in the heart in a procedure similar to the typical placement procedures for cardiac right ventricle leads. Electrode lead **109** is placed in the heart using the standard cardiac lead devices which include introducers, guide catheters, guidewires, and/or stylets. Electrode lead **109** is inserted into the clavicle vein, through the superior vena cava, through the right atrium and down into the right ventricle. Electrode lead **109** is positioned under fluoroscopy into the location the clinician has determined is clinically optimal and logistically practical for fixating the electrode lead **109.** Under fluoroscopy, the active fixation helix **116** is advanced and screwed into the cardiac tissue to secure electrode lead **109** onto the septum. The electrode lead **108** is placed in the right atrium using an active fixation helix **118.** The distal tip electrode **118** is used to both provide pacing and motion sensing of the right atrium.

Yet another type of medical device and system in which the subject segmented electrode structures find use is vision restoration devices and systems, e.g., devices and systems that include implantable photodetector elements that convert detected light to electrical signals, e.g., for stimulating the optic nerve. For example, integrated circuits and photosensors, e.g., photovoltaic cells, can be coupled to segmented electrode structures of embodiments of the invention. Representative implantable vision restoration devices and systems in which the segmented electrode structures may be incorporated include, but are not limited to those devices and systems described in: U.S. Patent Nos. 4,628,933; 5,042,223; 5,397,350; and 6,230,057; as well as in Published PCT Application Publication Nos. WO 01/74444 titled "Multi-Phasic Microphotodetector Retinal Implant With Variable Voltage And Current Capability And Apparatus For Insertion"; WO 01/83026 titled "Artificial Retina Device With Stimulating And Ground Return Electrodes Disposed On Opposite Sides Of The Neuroretina And Method Of Attachment' ; WO 03/002190 titled "Methods For Improving Damaged Retinal Cell Function; WO 03/002070 titled "Methods For Improving Damaged Retinal Cell Function Using Physical And/Or Mechanical Stimulation"; WO 2004/071338 titled "Implantable Device Using Diamond-Like Carbon Coating"; WO 2004/112893 titled "Implant Instrument"; WO 2005/004985 titled "Treatment Of Degenerative Retinal Disease Via Electrical Stimulation Of Surface Stuctures"; WO 2005/004985 titled "Device For Treatment Of Degenerative Retinal Disease Via Electrical Stimulation Of Surface Stuctures Of The Eyeball"; and WO 2005/110326 titled "Mechanically Activated Objects For Treatment Of Degenerative Retinal Disease."

### KITS

Also provided are kits that include the subject segmented electrode structures, as part of one or more components of an implantable device or system, such as the devices and systems reviewed above. In certain embodiments, the kits further include at least a control unit, e.g., in the form of a pacemaker can. In certain of these embodiments, the structure and control unit may be electrically coupled by an elongated conductive member. In certain embodiments, the segmented electrode sealed structure may be present in a lead, such as a cardiovascular lead.

In certain embodiments of the subject kits, the kits will further include instructions for using the subject devices or elements for obtaining the same (e.g., a website URL directing the user to a webpage which provides the instructions), where these instructions are typically printed on a substrate, which substrate may be one or more of: a package insert, the packaging, reagent containers and the like. In the subject kits, the one or more components are present in the same or different containers, as may be convenient or desirable.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

Accordingly, the preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope of present invention is embodied by the appended claims.

## Claims

1. An implantable addressable segmented electrode structure comprising:
an integrated circuit (2;22;42;52; 2;75;81;91;162;171;192;201,202; 403); and
at least one elongated conductive member (405,407), wherein the integrated circuit is electrically coupled to the at least one elongated conductive member (405,407);
two or more electrodes (1;21;41;51;61;71;82;191409A-409D) coupled to said integrated circuit, **characterized in that** each of said electrodes is individually addressable by the integrated circuit and that said electrodes are arranged circumferentially around said integrated circuit (403).

2. The implantable addressable segmented electrode structure of claim 1, wherein said structure is dimensioned to fit within a lead.

3. The implantable addressable segmented electrode structure of claim 2, the electrodes having an outer curved surface matched to the configuration of the lead body.

4. The implantable addressable segmented electrode structure of claim 1 or 2, the integrated circuit (403) comprising attachment tabs (417) and each electrode (409A, 409b, 409C, 409D) comprising a juncture (411), wherein the attachment tabs (417) are configured for positioning into a respective juncture (411).

5. The implantable addressable segmented electrode structure of any of the preceding claims, and wherein said integrated circuit and elongated conductive member are conductively connected to each other by a flexible conductive member (401).

6. The implantable addressable segmented electrode structure of claim 5, wherein the integrated circuit (403) comprises an attachment tab (417) and wherein the flexible conductive member (401) is attached to the attachment tab (417), preferably welded to the attachment tab (417).

7. The implantable addressable segmented electrode structure of any of the previous claims, comprising:
- an integrated circuit (403) with six attachment tabs (417) and four segmented electrodes (409A-D) arranged circumferentially around the integrated circuit, each segmented electrode welded to a respective attachment tab (417), wherein the integrated circuit (403) multiplexes between any of the four electrodes and either of the remaining attachment tabs.

8. Implantable addressable segmented electrode structure of claim 7, further comprising two conductors, each conductor welded to a respective remaining attachment tab of each of the at least one IC electrode structures, wherein, preferably, each integrated circuit multiplexes between any of the four electrodes and either of the two conductors in a lead and/or the attachment tabs and the electrodes are made of substantially the same material and/or the electrodes are embedded in a ring of polymer, the structure preferably comprising two structures.

9. The implantable addressable segmented electrode structure of any of the previous claims, wherein the electrodes are configured to pace and sense independently with the use of the integrated circuit (IC), preferably a multiplexing circuit.

10. The implantable addressable segmented electrode structure of any of the previous claims, wherein the electrodes are supported by a polymer (306), for instance PEEK, PEKK, polyamide, ETFE, urethane, ceramic material, alumina, or silicon carbide.

11. The implantable addressable segmented electrode structure of claim 1, wherein said electrodes are substantially aligned or electrodes are substantially staggered, and/or wherein the integrated circuit is configured to selectively activate one or more of the electrodes of the segmented structure so as to direct electrical current to only that tissue that needs to be excited; and/or wherein said integrated circuit is electrically coupled to at least one elongated conductive member in a medical carrier, for example wherein said integrated circuit is electrically coupled to a single elongated conductive member wherein said integrated circuit is electrically coupled to two elongated conductive members, or wherein said elongated conductive member is electrically coupled to at least one control unit, preferably wherein said control unit is present in a pacemaker can; wherein said integrated circuit is less than about 1 mm from said electrodes, preferably wherein said integrated circuit is less than about 20 mm from said electrodes, or wherein said integrated circuit comprises said electrodes, or wherein said structure comprises electrodes that are interdigitated, or herein said structure comprises electrodes of at least two different sizes, or wherein said structure comprises electrodes of about the same size, or wherein said structure comprises four electrodes, or wherein said structure comprises three electrodes, or wherein said structure is dimensioned to fit within an implant, for example, or wherein each electrode has a surface area ranging from about 0.1 mm² to about 15 mm² preferably wherein each electrode has a surface area ranging from about 0.5 mm² to about 10 mm², more preferably wherein each electrode has a surface area that is about 1.3 mm²; and/or wherein integrated circuit, electrodes and at least one elongated conductive member are electrically coupled to each other in a manner that imparts fatigue resistance to said lead assembly, preferably by one of the following: wherein at least two of said integrated circuit, electrodes and elongated conductive member are electrically coupled to each other in a manner that minimizes mechanical stress on said structure;
wherein at least two of said integrated circuit, electrodes and elongated conductive member are conductively connected to each other by a liquid member; wherein at least two of said integrated circuit, electrodes and elongated conductive member are conductively connected to each other by a coil conductive member; wherein at least two of said integrated circuit, electrodes and elongated conductive member are conductively connected to each other by a spherical conductive member; and or
wherein said electrodes have a curved configuration, or wherein said electrodes are flexible, preferably wherein said electrodes comprises one or more hairpin turns, or wherein said electrodes have a helical configuration, or
wherein said integrated circuit comprises at least one through hole, preferably wherein said integrated circuit comprises at least two through holes; and or
wherein said integrated circuit has a non-rectangular configuration, preferably wherein said integrated circuit has a curvilinear configuration, more preferably wherein said integrated circuit is disc-shaped.

12. The implantable addressable segmented electrode structure according to Claim 1, wherein said integrated circuit is a hermetically sealed integrated circuit, wherein said hermetically sealed integrated circuit preferably comprises:
an in vivo corrosion resistant integrated circuit holder having at least one feedthrough;
at least one integrated circuit present in said holder; and
a sealing layer;
wherein said sealing layer and holder are configured to define a hermetically sealed volume in which said at least one integrated circuit is present.

13. A system comprising:
an implantable addressable segmented electrode structure according to any of the claims 1- 12 and
a control unit wherein said structure and control unit are electrically coupled by at least one elongated conductive member, the lead preferably being a cardiovascular lead and the control unit preferably being a pacemaker can.

14. A kit comprising:
an implantable addressable segmented electrode structure according to any of the claims 1-12; and
a control unit wherein said kit preferably further includes an elongated conductive member, and wherein said structure is preferably present in a lead, preferably wherein said lead is a cardiovascular lead and wherein said control unit is preferably present in a pacemaker can.

## Patentansprüche

1. Implantierbare adressierbare segmentierte Elektrodenstruktur, umfassend:
eine integrierte Schaltung (2; 22; 42; 52; 2; 75; 81; 91; 162; 171; 192; 201, 202; 403); und
mindestens ein längliches leitfähiges Element (405, 407), wobei die integrierte Schaltung mit dem mindestens einen länglichen leitfähigen Element (405, 407) elektrisch verbunden ist;
zwei oder mehr Elektroden (1; 21; 41; 51; 61; 71; 82; 191; 409A - 409D), die mit der integrierten Schaltung gekoppelt sind, **dadurch gekennzeichnet, dass** jede der Elektroden einzeln durch die integrierte Schaltung adressierbar ist und dass die Elektroden in Umfangsrichtung um die integrierte Schaltung (403) herum angeordnet sind.

2. Implantierbare adressierbare segmentierte Elektrodenstruktur nach Anspruch 1, wobei die Struktur derart bemessen ist, dass sie in ein Kabel passt.

3. Implantierbare adressierbare segmentierte Elektrodenstruktur nach Anspruch 2, wobei die Elektroden eine gekrümmte Außenseite aufweisen, die der Konfiguration des Kabelkörpers angepasst ist.

4. Implantierbare adressierbare segmentierte Elektrodenstruktur nach Anspruch 1 oder 2, wobei die integrierte Schaltung (403) Befestigungsnasen (417) umfasst und jede Elektrode (409A, 409B, 409C, 409D) eine Verbindungsstelle (411) umfasst, wobei die Befestigungsnasen (417) zur Positionierung in einer jeweiligen Verbindungsstelle (411) konfiguriert sind.

5. Implantierbare adressierbare segmentierte Elektrodenstruktur nach einem der vorhergehenden Ansprüche, wobei außerdem die integrierte Schaltung und das längliche leitfähige Element durch ein flexibles leitfähiges Element (401) leitfähig miteinander verbunden sind.

6. Implantierbare adressierbare segmentierte Elektrodenstruktur nach Anspruch 5, wobei die integrierte Schaltung (403) eine Befestigungsnase (417) aufweist und wobei das flexible leitfähige Element (401) an der Befestigungsnase (417) befestigt ist, vorzugsweise an der Befestigungsnase (417) angeschweißt ist.

7. Implantierbare adressierbare segmentierte Elektrodenstruktur nach einem der vorhergehenden Ansprüche, umfassend:
- eine integrierte Schaltung (403) mit sechs Befestigungsnasen (417) und vier segmentierte Elektroden (409A-D), die in Umfangsrichtung um die integrierte Schaltung herum angeordnet sind, wobei jede segmentierte Elektrode an einer jeweiligen Befestigungsnase (417) angeschweißt ist, wobei die integrierte Schaltung (403) zwischen einer der vier Elektroden und einer der verbleibenden Befestigungsnasen multiplext.

8. Implantierbare adressierbare segmentierte Elektrodenstruktur nach Anspruch 7, welche ferner zwei Leiter umfasst, wobei jeder Leiter an eine entsprechende verbleibende Befestigungsnase jeder der mindestens einen IC-Elektrodenstruktur(en) angeschweißt ist, wobei vorzugsweise jede integrierte Schaltung zwischen einer der vier Elektroden und einem der beiden Leiter in einem Kabel multiplext, und/oder die Befestigungsnasen und die Elektroden aus im Wesentlichen dem gleichen Material bestehen und/oder die Elektroden in einen Polymerring eingebettet sind, wobei die Struktur vorzugsweise zwei Strukturen umfasst.

9. Implantierbare adressierbare segmentierte Elektrodenstruktur nach einem der vorhergehenden Ansprüche, wobei die Elektroden unter Verwendung der integrierten Schaltung (IC), vorzugsweise einer Multiplex-Schaltung, dafür konfiguriert sind, unabhängig Schrittmacher- und Messfunktion auszuführen.

10. Implantierbare adressierbare segmentierte Elektrodenstruktur nach einem der vorhergehenden Ansprüche, wobei die Elektroden durch ein Polymer (306), beispielsweise PEEK, PEKK, Polyamid, ETFE, Urethan, Keramikmaterial, Aluminiumoxid oder Siliziumcarbid gehalten werden.

11. Implantierbare adressierbare segmentierte Elektrodenstruktur nach Anspruch 1, wobei die Elektroden im Wesentlichen zueinander ausgerichtet sind oder die Elektroden im Wesentlichen versetzt angeordnet sind, und/oder wobei die integrierte Schaltung dafür konfiguriert ist, selektiv eine oder mehrere der Elektroden der segmentierten Struktur zu aktivieren, um elektrischen Strom nur zu demjenigen Gewebe zu leiten, das angeregt werden muss; und/oder wobei die integrierte Schaltung elektrisch mit mindestens einem länglichen leitfähigen Element in einem medizinischen Träger gekoppelt ist, wobei beispielsweise die integrierte Schaltung elektrisch mit einem einzelnen länglichen leitfähigen Element gekoppelt ist, wobei die integrierte Schaltung elektrisch mit zwei länglichen leitfähigen Elementen gekoppelt ist oder wobei das längliche leitfähige Element elektrisch mit mindestens einer Steuereinheit gekoppelt ist, wobei vorzugsweise die Steuereinheit in einem Schrittmachergehäuse angeordnet ist; wobei die integrierte Schaltung weniger als etwa 1 mm von den Elektroden entfernt ist, wobei vorzugsweise die integrierte Schaltung weniger als etwa 20 mm von den Elektroden entfernt ist, oder wobei die integrierte Schaltung die Elektroden umfasst, oder wobei die Struktur miteinander verschränkte Elektroden umfasst, oder wobei hierbei die Struktur Elektroden mit mindestens zwei unterschiedlichen Größen umfasst, oder wobei die Struktur Elektroden von etwa der gleichen Größe umfasst, oder wobei die Struktur vier Elektroden umfasst, oder wobei die Struktur drei Elektroden umfasst, oder wobei die Struktur derart dimensioniert ist, dass sie beispielsweise in ein Implantat passt, oder wobei jede Elektrode eine Oberfläche im Bereich zwischen etwa 0,1 mm² und etwa 15 mm² aufweist, wobei vorzugsweise jede Elektrode eine Oberfläche im Bereich zwischen etwa 0,5 mm² und etwa 10 mm² aufweist, wobei bevorzugter jede Elektrode eine Oberfläche von ungefähr 1,3 mm² aufweist; und/oder wobei integrierte Schaltung, Elektroden und mindestens ein längliches leitfähiges Element elektrisch miteinander in einer Weise gekoppelt sind, die der Kabelanordnung Ermüdungsbeständigkeit verleiht, vorzugsweise durch eines der folgenden Merkmale: wobei mindestens zwei der Elemente integrierte Schaltung, Elektroden und längliches leitfähiges Element elektrisch so miteinander gekoppelt sind, dass die mechanische Belastung der Struktur minimiert wird;
wobei mindestens zwei der Elemente integrierte Schaltung, Elektroden und längliches leitfähiges Element durch ein flüssiges Element leitfähig miteinander verbunden sind; wobei mindestens zwei der Elemente integrierte Schaltung, Elektroden und längliches leitfähiges Element durch ein gewundenes leitfähiges Element leitfähig miteinander verbunden sind; wobei mindestens zwei der Elemente integrierte Schaltung, Elektroden und längliches leitfähiges Element durch ein kugelförmiges leitfähiges Element leitfähig miteinander verbunden sind; und/oder
wobei die Elektroden eine gebogene Konfiguration aufweisen oder wobei die Elektroden flexibel sind, wobei die Elektroden vorzugsweise einen oder mehrere Haarnadelbögen aufweisen oder wobei die Elektroden eine schraubenförmige Konfiguration aufweisen, oder
wobei die integrierte Schaltung mindestens ein Durchgangsloch umfasst, wobei die integrierte Schaltung vorzugsweise mindestens zwei Durchgangslöcher umfasst; und/oder
wobei die integrierte Schaltung eine nicht rechteckige Konfiguration aufweist, wobei die integrierte Schaltung vorzugsweise eine gekrümmte Konfiguration aufweist, wobei die integrierte Schaltung bevorzugter scheibenförmig ist.

12. Implantierbare adressierbare segmentierte Elektrodenstruktur nach Anspruch 1, wobei die integrierte Schaltung eine hermetisch abgedichtete integrierte Schaltung ist, wobei die hermetisch abgedichtete integrierte Schaltung vorzugsweise umfasst:
einen *in vivo* korrosionsbeständigen Halter für die integrierte Schaltung mit mindestens einer Durchführung,
mindestens eine in dem Halter angeordnete integrierte Schaltung und
eine Versiegelungsschicht,
wobei die Versiegelungsschicht und der Halter so konfiguriert sind, dass sie ein hermetisch abgedichtetes Volumen bestimmen, in dem die mindestens eine integrierte Schaltung angeordnet ist.

13. System, umfassend:
eine implantierbare adressierbare segmentierte Elektrodenstruktur nach einem der Ansprüche 1 - 12 und
eine Steuereinheit, wobei die Struktur und die Steuereinheit durch mindestens ein längliches leitfähiges Element elektrisch gekoppelt sind, wobei das Kabel vorzugsweise ein kardiovaskuläres Kabel ist und die Steuereinheit vorzugsweise ein Schrittmachergehäuse ist.

14. Kit, umfassend:
eine implantierbare adressierbare segmentierte Elektrodenstruktur nach einem der Ansprüche 1 - 12 und
eine Steuereinheit, wobei das Kit ferner vorzugsweise ein längliches leitfähiges Element umfasst und wobei die Struktur vorzugsweise in einem Kabel angeordnet ist, wobei das Kabel vorzugsweise ein kardiovaskuläres Kabel ist und wobei die Steuereinheit vorzugsweise in einem Schrittmachergehäuse angeordnet ist.

## Revendications

1. Structure d'électrode segmentée adressable implantable comprenant :
un circuit intégré (2 ; 22 ; 42 ; 52 ; 2 ; 75 ; 81 ; 91 ; 162 ; 171 ; 192 ; 201, 202 ; 403) ; et
au moins un élément conducteur allongé (405, 407), dans laquelle le circuit intégré est électriquement couplé à l'au moins un élément conducteur allongé (405, 407) ;
deux électrodes ou plus (1 ; 21 ; 41 ; 51 ; 61 ; 71 ; 82 ; 191409A-409D) couplées audit circuit intégré, **caractérisée en ce que** chacune desdites électrodes est individuellement adressable par le circuit intégré et que lesdites électrodes sont agencées de façon circonférentielle autour dudit circuit intégré (403).

2. Structure d'électrode segmentée adressable implantable selon la revendication 1, ladite structure étant dimensionnée pour s'ajuster à l'intérieur d'un conduit.

3. Structure d'électrode segmentée adressable implantable selon la revendication 2, les électrodes ayant une surface courbée extérieure adaptée à la configuration du corps de conduit.

4. Structure d'électrode segmentée adressable implantable selon la revendication 1 ou 2, le circuit intégré (403) comprenant des languettes de fixation (417) et chaque électrode (409A, 409b, 409C, 409D) comprenant une jonction (411), dans laquelle les languettes de fixation (417) sont configurées pour un positionnement dans une jonction respective (411).

5. Structure d'électrode segmentée adressable implantable selon l'une quelconque des revendications précédentes, et dans laquelle lesdits circuit intégré et élément conducteur allongé sont reliés l'un à l'autre de façon conductrice par un élément conducteur flexible (401).

6. Structure d'électrode segmentée adressable implantable de la revendication 5, dans laquelle le circuit intégré (403) comprend une languette de fixation (417) et dans laquelle l'élément conducteur flexible (401) est fixé à la languette de fixation (417), de préférence soudé à la languette de fixation (417).

7. Structure d'électrode segmentée adressable implantable selon l'une quelconque des revendications précédentes, comprenant :
- un circuit intégré (403) avec six languettes de fixation (417) et quatre électrodes segmentées (409A-D) agencées de façon circonférentielle autour du circuit intégré, chaque électrode segmentée étant soudée à une languette de fixation (417) respective, dans laquelle le circuit intégré (403) effectue un multiplexage entre l'une quelconque des quatre électrodes et l'une des languettes de fixation restantes.

8. Structure d'électrode segmentée adressable implantable selon la revendication 7, comprenant en outre deux conducteurs, chaque conducteur étant soudé à une languette de fixation restante respective de chacune des au moins une structure d'électrode à CI, dans laquelle, de préférence, chaque circuit intégré effectue un multiplexage entre l'une quelconque des quatre électrodes et l'un des deux conducteurs dans un conduit et/ou les languettes de fixation et les électrodes sont constituées de sensiblement le même matériau et/ou les électrodes sont incorporées dans un anneau de polymère, la structure comprenant de préférence deux structures.

9. Structure d'électrode segmentée adressable implantable selon l'une quelconque des revendications précédentes, dans laquelle les électrodes sont configurées pour stimuler et détecter indépendamment par l'utilisation du circuit intégré (IC), de préférence un circuit de multiplexage.

10. Structure d'électrode segmentée adressable implantable selon l'une quelconque des revendications précédentes, dans laquelle les électrodes sont soutenues par un polymère (306), par exemple le PEEK, le PEKK, le polyamide, l'ETFE, l'uréthane, un matériau céramique, l'alumine ou le carbure de silicium.

11. Structure d'électrode segmentée adressable implantable selon la revendication 1, dans laquelle lesdites électrodes sont sensiblement alignées ou les électrodes sont sensiblement échelonnées, et/ou dans laquelle le circuit intégré est configuré pour activer sélectivement une ou plusieurs des électrodes de la structure segmentée de manière à diriger un courant électrique uniquement vers le tissu qui doit être stimulé ; et/ou dans laquelle ledit circuit intégré est électriquement couplé à au moins un élément conducteur allongé dans un support médical, par exemple dans laquelle ledit circuit intégré est électriquement couplé à un élément conducteur allongé unique, dans laquelle ledit circuit intégré est électriquement couplé à deux éléments conducteurs allongés, ou dans laquelle ledit élément conducteur allongé est électriquement couplé à au moins une unité de commande, de préférence dans laquelle ladite unité de commande est présente dans un boîtier de stimulateur cardiaque ; dans laquelle ledit circuit intégré est à moins d'environ 1 mm desdites électrodes, de préférence dans laquelle ledit circuit intégré est à moins d'environ 20 mm desdites électrodes, ou dans laquelle ledit circuit intégré comprend lesdites électrodes, ou dans laquelle ladite structure comprend des électrodes qui sont interdigitées, ou dans laquelle ladite structure comprend des électrodes d'au moins deux tailles différentes, ou dans laquelle ladite structure comprend des électrodes d'environ la même taille, ou dans laquelle ladite structure comprend quatre électrodes, ou dans laquelle ladite structure comprend trois électrodes, ou dans laquelle ladite structure est dimensionnée pour s'ajuster à l'intérieur d'un implant, par exemple, ou dans laquelle chaque électrode a une surface dans la plage d'environ 0,1 mm² à environ 15 mm², de préférence dans laquelle chaque électrode a une surface dans la plage d'environ 0,5 mm² à environ 10 mm², plus préférablement dans laquelle chaque électrode a une surface qui est d'environ 1,3 mm² ; et/ou dans laquelle le circuit intégré, les électrodes et au moins un élément conducteur allongé sont électriquement couplés les uns aux autres d'une manière qui confère une résistance à la fatigue audit ensemble de conduit, de préférence par l'un des suivants : dans laquelle au moins deux desdits circuit intégré, électrodes et élément conducteur allongé sont électriquement couplés les uns aux autres d'une manière qui réduit au minimum l'effort mécanique sur ladite structure ;
dans laquelle au moins deux desdits circuit intégré, électrodes et élément conducteur allongé sont reliés de façon conductrice les uns aux autres par un élément liquide ; dans laquelle au moins deux desdits circuit intégré, électrodes et élément conducteur allongé sont reliés de façon conductrice les uns aux autres par un élément conducteur enroulé ; dans laquelle au moins deux desdits circuit intégré, électrodes et élément conducteur allongé sont reliés de façon conductrice les uns aux autres par un élément conducteur sphérique ; et/ou
dans laquelle lesdites électrodes ont une configuration courbée, ou dans laquelle lesdites électrodes sont flexibles, de préférence dans laquelle lesdites électrodes comprennent un ou plusieurs tournants en épingle à cheveux, ou dans laquelle lesdites électrodes ont une configuration hélicoïdale, ou
dans laquelle ledit circuit intégré comprend au moins un trou de passage, de préférence dans laquelle ledit circuit intégré comprend au moins deux trous de passage ; et/ou dans laquelle ledit circuit intégré a une configuration non rectangulaire, de préférence dans laquelle ledit circuit intégré a une configuration curviligne, plus préférablement dans laquelle ledit circuit intégré étant en forme de disque.

12. Structure d'électrode segmentée adressable implantable selon la revendication 1, dans laquelle ledit circuit intégré étant un circuit intégré hermétiquement scellé, dans laquelle ledit circuit intégré hermétiquement scellé comprend de préférence :
un support de circuit intégré résistant à la corrosion in vivo ayant au moins une traversée ;
au moins un circuit intégré présent dans ledit support ; et
une couche d'étanchéité ;
dans laquelle ladite couche d'étanchéité et le support sont configurés pour définir un volume hermétiquement scellé dans lequel ledit au moins un circuit intégré est présent.

13. Système comprenant :
une structure d'électrode segmentée adressable implantable selon l'une quelconque des revendications 1 à 12 et
une unité de commande, dans laquelle lesdites structure et unité de commande sont électriquement couplées par au moins un élément conducteur allongé, le conduit étant de préférence un conduit cardiovasculaire et l'unité de commande étant de préférence un boîtier de stimulateur cardiaque.

14. Kit comprenant :
une structure d'électrode segmentée adressable implantable selon l'une quelconque des revendications 1 à 12 ; et
une unité de commande, dans laquelle ledit kit comprend en outre de préférence un élément conducteur allongé, et dans laquelle ladite structure est de préférence présente dans un conduit, de préférence dans laquelle ledit conduit est un conduit cardiovasculaire et dans laquelle ladite unité de commande étant de préférence présente dans un boîtier de stimulateur cardiaque.
